(19) Europäisches Patentamt
European Patent Office
Office européen des brevets

(11) EP 4 151 672 A1

(12) # EUROPEAN PATENT APPLICATION
published in accordance with Art. 153(4) EPC

(43) Date of publication:
**22.03.2023 Bulletin 2023/12**

(21) Application number: **21805023.5**

(22) Date of filing: **11.05.2021**

(51) International Patent Classification (IPC):
***C08G 73/12*** *(2006.01)* ***C08G 73/18*** *(2006.01)*
***C09D 179/04*** *(2006.01)* ***C09D 179/08*** *(2006.01)*
***C09J 179/08*** *(2006.01)*

(52) Cooperative Patent Classification (CPC):
**C07D 209/48; C08G 61/12; C08G 73/12; C08G 73/18; C09D 7/63; C09D 161/00; C09D 179/04; C09D 179/08; C09J 11/06; C09J 161/00; C09J 179/08**

(86) International application number:
**PCT/JP2021/017951**

(87) International publication number:
**WO 2021/230254 (18.11.2021 Gazette 2021/46)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **11.05.2020 JP 2020083262**

(71) Applicants:
- **Daikin Industries, Ltd.**
**Osaka-shi, Osaka 530-0001 (JP)**
- **NATIONAL UNIVERSITY CORPORATION, IWATE UNIVERSITY**
**Morioka-shi, Iwate 020-8550 (JP)**

(72) Inventors:
- **OISHI, Yoshiyuki**
**Morioka-shi, Iwate 020-8550 (JP)**
- **NOGUCHI, Tsuyoshi**
**Osaka-shi, Osaka 530-8323 (JP)**

(74) Representative: **Hoffmann Eitle**
**Patent- und Rechtsanwälte PartmbB**
**Arabellastraße 30**
**81925 München (DE)**

(54) **AMIDE COMPOUND, NITROGEN-CONTAINING HETEROCYCLIC COMPOUND, AND CROSSLINKED PRODUCT**

(57) Provided are an amide compound and a nitrogen-containing heterocyclic ring-containing compound which have a specific repeating unit and a crosslinking site represented by Formula (2):

$$-C{\equiv}CX^1$$

at a molecular end.

EP 4 151 672 A1

## Description

TECHNICAL FIELD

The present disclosure relates to an amide compound, a nitrogen-containing heterocyclic ring-containing compound, and a crosslinked product.

BACKGROUND ART

Non Patent Document 1 and Patent Document 1 describe that polyimide and polybenzimidazole have the highest level of heat resistance, strength, and chemical stability as polymer materials, and used in various fields as heat-resistant fibers, molded articles, coating varnishes, and the like.

RELATED ART

NON PATENT DOCUMENT

Non Patent Document 1: Japan Polyimide / Aromatic Polymer Research Group, "Newly Revised Latest Polyimides -Basics and Applications-", NTS Co., Ltd., published August 25, 2010, pp. 222 to 230.

PATENT DOCUMENT

Patent Document 1: Japanese Patent Laid-Open No. 9-208699

SUMMARY OF THE INVENTION

PROBLEM TO BE SOLVED BY THE INVENTION

It is an object of the present disclosure to provide an amide compound that can obtain, by crosslinking, a crosslinked product exhibiting elastomeric characteristics at a high temperature and furthermore in a wide temperature range, and a crosslinked product having excellent heat resistance by crosslinking.

Further, it is an object of the present disclosure to provide a nitrogen-containing heterocyclic ring-containing compound that can obtain, by crosslinking, a crosslinked product exhibiting elastomeric characteristics at a high temperature and furthermore in a wide temperature range, and a crosslinked product having excellent heat resistance by crosslinking.

Further, it is an object of the present disclosure to provide a crosslinked product exhibiting elastomeric characteristics at a high temperature and furthermore in a wide temperature range, and a crosslinked product having excellent heat resistance.

MEANS FOR SOLVING THE PROBLEM

According to the present disclosure, there is provided an amide compound including a repeating unit represented by Formula (1) and a crosslinking site represented by Formula (2) at a molecular end.

Formula (1):

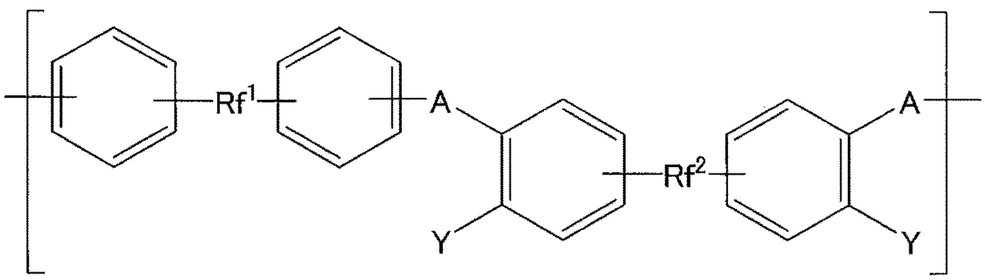

(In Formula (1),

Rf[1] represents a divalent fluorinated organic group,
$Rf^2$ represents a single bond, $-SO_2-$, -O-, -CO-, a divalent non-fluorinated organic group, or a divalent fluorinated organic group,
A represents an amide bond,
each Y independently represents $-COR^1$ or $-NHR^2$,
each $R^1$ independently represents OH, an optionally-substituted linear or branched chain alkoxy group, an optionally-substituted aromatic oxy group, or a halogen atom, and
each $R^2$ independently represents H or a monovalent organic group.)

Formula (2): $-C \equiv CX^1$

(In Formula (2), $X^1$ represents H or a monovalent organic group.)

The average degree of polymerization of the repeating unit represented by Formula (1) is preferably 2 to 10.

The repeating unit represented by Formula (1) is preferably a repeating unit represented by Formula (1a).

Formula (1a):

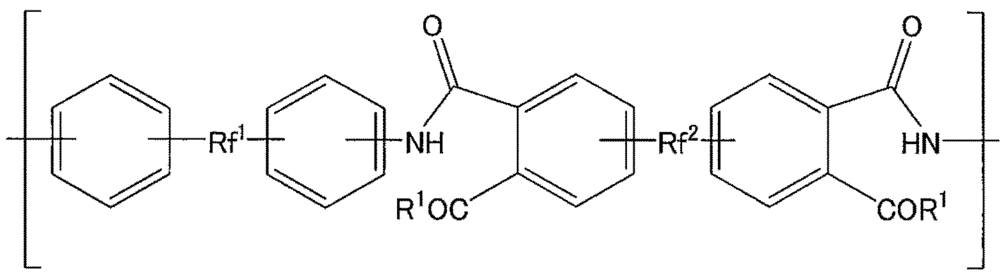

(In Formula (1a),

$Rf^1$ represents a divalent fluorinated organic group,
$Rf^2$ represents a single bond, $-SO_2-$, -O-, -CO-, a divalent non-fluorinated organic group, or a divalent fluorinated organic group, and
each $R^1$ independently represents OH, an optionally-substituted linear or branched chain alkoxy group, an optionally-substituted aromatic oxy group, or a halogen atom.)

The repeating unit represented by Formula (1) is preferably a repeating unit represented by Formula (1b).

Formula (1b):

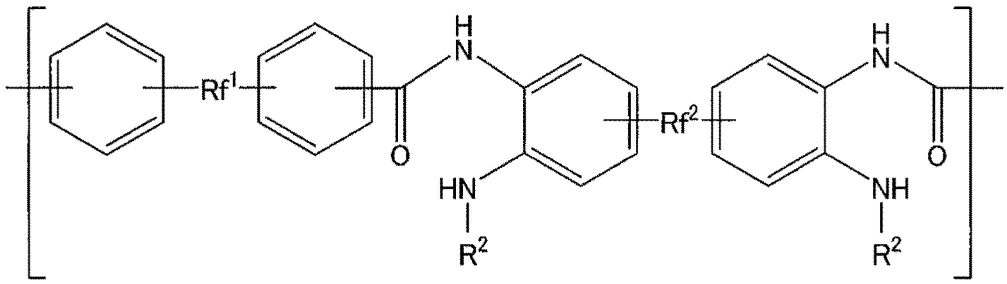

(In Formula (1b),

$Rf^1$ represents a divalent fluorinated organic group,
$Rf^2$ represents a single bond, $-SO_2-$, -O-, -CO-, a divalent non-fluorinated organic group, or a divalent fluorinated organic group, and
each $R^2$ independently represents H or a monovalent organic group.)
$Rf^1$ is preferably a perfluoroalkylene group having 4 or more carbon atoms.

Further, according to the present disclosure, there is provided a nitrogen-containing heterocyclic ring-containing compound including a repeating unit represented by Formula (3) and a crosslinking site represented by Formula (2) at a molecular end.

Formula (3):

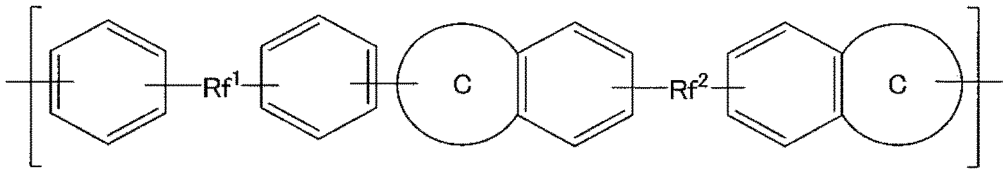

(In Formula (3),

$Rf^1$ represents a divalent fluorinated organic group,
$Rf^2$ represents a single bond, $-SO_2-$, -O-, -CO-, a divalent non-fluorinated organic group, or a divalent fluorinated organic group, and
a ring C represents an optionally-substituted imide ring or benzimidazole ring.)

Formula (2): $-C\equiv CX^1$

(In Formula (2), $X^1$ represents H or a monovalent organic group.)
The average degree of polymerization of the repeating unit represented by Formula (3) is preferably 2 to 10.
The repeating unit represented by Formula (3) is preferably a repeating unit represented by Formula (3a).

Formula (3a):

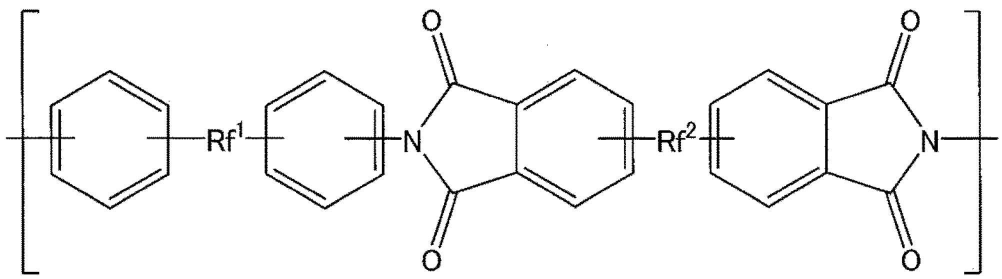

(In Formula (3a),

$Rf^1$ represents a divalent fluorinated organic group, and
$Rf^2$ represents a single bond, $-SO_2-$, -O-, -CO-, a divalent non-fluorinated organic group, or a divalent fluorinated organic group.)

The repeating unit represented by Formula (3) is preferably a repeating unit represented by Formula (3b).

Formula (3b):

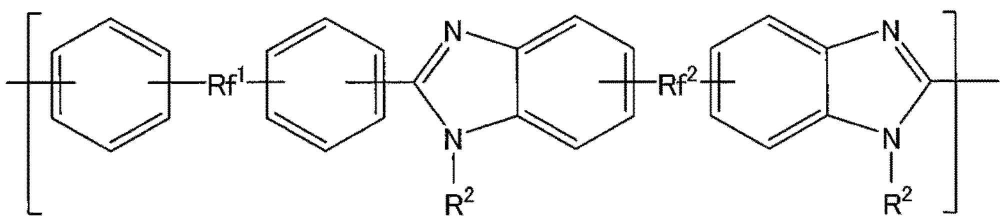

(In Formula (3b),

$Rf^1$ represents a divalent fluorinated organic group,
$Rf^2$ represents a single bond, $-SO_2-$, -O-, -CO-, a divalent non-fluorinated organic group, or a divalent fluorinated organic group, and
each $R^2$ independently represents H or a monovalent organic group.)
$Rf^1$ is preferably a perfluoroalkylene group having 4 or more carbon atoms.

Further, according to the present disclosure, there is provided an adhesive including the above-mentioned amide compound or the above-mentioned nitrogen-containing heterocyclic ring-containing compound.
Further, according to the present disclosure, there is provided a coating material including the above-mentioned amide compound or the above-mentioned nitrogen-containing heterocyclic ring-containing compound.
Further, according to the present disclosure, there is provided a crosslinked product obtained by crosslinking the above-mentioned amide compound or the above-mentioned nitrogen-containing heterocyclic ring-containing compound.
The above-mentioned crosslinked product can be suitably utilized as a member used in a semiconductor manufacturing apparatus.

EFFECTS OF INVENTION

According to the present disclosure, there can be provided an amide compound that can obtain, by crosslinking, a crosslinked product exhibiting elastomeric characteristics at a high temperature and furthermore in a wide temperature range, and a crosslinked product having excellent heat resistance by crosslinking.
Further, according to the present disclosure, there can be provided a nitrogen-containing heterocyclic ring-containing compound that can obtain, by crosslinking, a crosslinked product exhibiting elastomeric characteristics at a high temperature and furthermore in a wide temperature range, and a crosslinked product having excellent heat resistance by crosslinking.
Further, according to the present disclosure, there can be provided a crosslinked product exhibiting elastomeric characteristics at a high temperature and furthermore in a wide temperature range, and a crosslinked product having excellent heat resistance.

DESCRIPTION OF EMBODIMENTS

Hereinafter, specific embodiments of the present disclosure will be described in detail, but the present disclosure is not limited to the following embodiments.

<Amide compound>

The amide compound of the present disclosure has a repeating unit represented by Formula (1) and a crosslinking site represented by Formula (2) at a molecular end.

Formula (1):

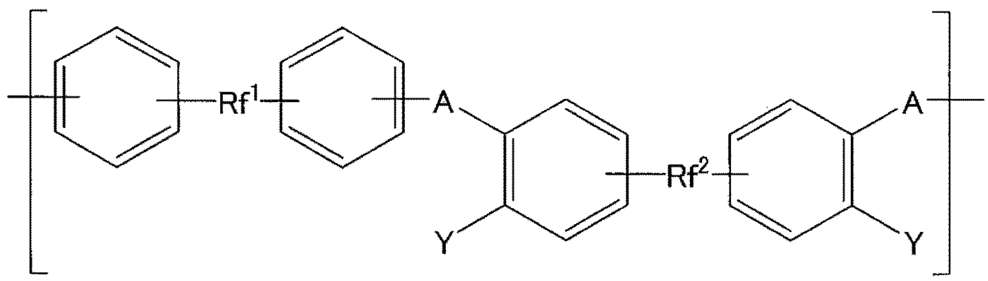

(In Formula (1),

$Rf^1$ represents a divalent fluorinated organic group,
$Rf^2$ represents a single bond, $-SO_2-$, -O-, -CO-, a divalent non-fluorinated organic group, or a divalent fluorinated organic group,

A represents an amide bond,
each Y independently represents $-COR^1$ or $-NHR^2$,
each $R^1$ independently represents OH, an optionally-substituted linear or branched chain alkoxy group, an optionally-substituted aromatic oxy group, or a halogen atom, and
each $R^2$ independently represents H or a monovalent organic group.)
$Rf^1$ represents a divalent fluorinated organic group. The fluorinated organic group is a divalent organic group having one or more fluorine atoms. From the perspective that it is possible to obtain a crosslinked product that exhibits elastomeric characteristics at a much higher temperature and furthermore in a much wider temperature range, and to obtain a crosslinked product that has much better heat resistance and that adheres much more firmly to a substrate, $Rf^1$ is preferably a linear or branched chain fluorinated alkylene group or a linear or branched chain fluorinated (poly)oxyalkylene group, and more preferably a linear or branched chain fluorinated alkylene group.

From the perspective that it is possible to obtain a crosslinked product that exhibits elastomeric characteristics at a much higher temperature and furthermore in a much wider temperature range, and to obtain a crosslinked product that has much better heat resistance and that adheres much more firmly to a substrate, the fluorinated alkylene group is preferably a perfluoroalkylene group. The perfluoroalkylene group is an alkylene group in which all of the hydrogen atoms have been substituted with fluorine atoms, and is an alkylene group that does not include a carbon atom-hydrogen atom bond in the molecule.

From the perspective that it is possible to obtain a crosslinked product that exhibits elastomeric characteristics at a much higher temperature and furthermore in a much wider temperature range, and to obtain a crosslinked product that has much better heat resistance and that adheres much more firmly to a substrate, the number of carbon atoms of the fluorinated alkylene group is preferably 1 to 80, more preferably 2 or more, further preferably 3 or more, particularly preferably 4 or more, and most preferably 5 or more, and is more preferably 60 or less, further preferably 40 or less, particularly preferably 20 or less, and most preferably 10 or less. The fluorinated alkylene group may have 4, 6 or 8 carbon atoms, and may be 6 or 8 carbon atoms.

From the perspective that it is possible to obtain a crosslinked product that exhibits elastomeric characteristics at a much higher temperature and furthermore in a much wider temperature range, and to obtain a crosslinked product that has much better heat resistance and that adheres much more firmly to a substrate, the fluorinated alkylene group preferably includes a unit represented by $-CRf^3{}_2-$ (in the formula each $Rf^3$ independently represents F, a linear or branched chain fluorinated alkyl group having 1 to 8 carbon atoms, or a linear or branched chain fluorinated alkoxy group having 1 to 8 carbon atoms). More preferably, the fluorinated alkylene group includes a unit represented by $-CF_2-$, $-CF(CF_3)-$, $-C(CF_3)_2-$, $-CF(OCF_3)-$, $-CF(CC_2F_5)-$, or $-CF(OC_3F_7)-$, further preferably a unit represented by $-CF_2-$, $-CF(CF_3)-$, $-CF(CCF_3)-$, $-CF(OC_2F_5)-$, or $-CF(CC_3F_7)-$, and particularly preferably a unit represented by $-CF_2-$.

From the perspective that it is possible to obtain a crosslinked product that exhibits elastomeric characteristics at a much higher temperature and furthermore in a much wider temperature range, and to obtain a crosslinked product that has much better heat resistance and that adheres much more firmly to a substrate, the fluorinated alkylene group is preferably a group represented by $-(CF_2-CF_2)_n-$, $-(CF_2-CF(CF_3))_n-$, $-(CF_2-CF(OCF_3))_n-$, $-(CF_2-CF(OC_2F_5))_n-$, or $-(CF_2-CF(OC_3F_7))_n-$ (in each formula, n represents an integer of 1 to 8), and more preferably is a group represented by $-(CF_2-CF_2)_n-$. From the viewpoint of the elastomeric characteristics, heat resistance, and adhesiveness of the obtained crosslinked product, n is preferably an integer of 2 or more, more preferably an integer of 2 to 4, and further preferably 3 or 4.

The fluorinated (poly)oxyalkylene group may be a fluorinated oxyalkylene group and a fluorinated polyoxyalkylene group. The fluorinated polyoxyalkylene group is a group to which two or more fluorinated oxyalkylene groups are bonded.

From the perspective that it is possible to obtain a crosslinked product that exhibits elastomeric characteristics at a much higher temperature and furthermore in a much wider temperature range, and to obtain a crosslinked product that has much better heat resistance and that adheres much more firmly to a substrate, the fluorinated (poly)oxyalkylene group is preferably a perfluoro(poly)oxyalkylene group. The perfluoro(poly)oxyalkylene group is a (poly)oxyalkylene group in which all of the hydrogen atoms have been substituted with fluorine atoms, and is a (poly)oxyalkylene group that does not include a carbon atom-hydrogen atom bond in the molecule.

From the perspective that it is possible to obtain a crosslinked product that exhibits elastomeric characteristics at a much higher temperature and furthermore in a much wider temperature range, and to obtain a crosslinked product that has much better heat resistance and that adheres much more firmly to a substrate, the number of carbon atoms of the fluorinated (poly)oxyalkylene group is preferably 1 to 80, more preferably 2 or more, and further preferably 3 or more, and is more preferably 60 or less, further preferably 40 or less, and particularly preferably 20 or less. The fluorinated oxyalkylene group may have 4, 6 or 8 carbon atoms.

From the perspective that it is possible to obtain a crosslinked product that exhibits elastomeric characteristics at a much higher temperature and furthermore in a much wider temperature range, and to obtain a crosslinked product that has much better heat resistance and that adheres much more firmly to a substrate, the fluorinated (poly)oxyalkylene

group preferably includes a unit represented by $-O-CRf^3_2-$ (in the formula each $Rf^3$ independently represents F, a linear or branched chain fluorinated alkyl group having 1 to 8 carbon atoms, or a linear or branched chain fluorinated alkoxy group having 1 to 8 carbon atoms). More preferably, the fluorinated (poly)oxyalkylene group includes a unit represented by $-O-CF_2-$, $-O-CF(CF_3)-$, $-O-CF(OCF_3)-$, $-O-CF(OC_2F_5)-$, or $-O-CF(OC_3F_7)-$.

From the perspective that it is possible to obtain a crosslinked product that exhibits elastomeric characteristics at a much higher temperature and furthermore in a much wider temperature range, and to obtain a crosslinked product that has much better heat resistance and that adheres much more firmly to a substrate, the fluorinated (poly)oxyalkylene group is preferably a group represented by $-(O-CF_2-CF_2)_n-$, $-(O-CF_2-CF(CF_3))_n-$, $-(O-CF_2-CF(OCF_3))_n-$, $-(O-CF_2-CF(OC_2F_5))_n-$, or $-(O-CF_2-CF(OC_3F_7))_n-$ (in each formula, n represents an integer of 1 to 8). From the viewpoint of the elastomeric characteristics, heat resistance, and adhesiveness of the obtained crosslinked product, n is preferably an integer of 2 or more, and more preferably an integer of 2 to 4.

$Rf^2$ represents a single bond, $-SO_2-$, -O-, -CO-, a divalent non-fluorinated organic group, or a divalent fluorinated organic group. From the perspective that it is possible to obtain a crosslinked product that exhibits elastomeric characteristics at a much higher temperature and furthermore in a much wider temperature range, and to obtain a crosslinked product that has much better heat resistance and that adheres much more firmly to a substrate, $Rf^2$ is preferably -O- or a divalent fluorinated organic group, and more preferably a divalent fluorinated organic group.

The non-fluorinated organic group is a divalent organic group that does not have a fluorine atom. The non-fluorinated organic group is preferably a linear or branched chain non-fluorinated alkylene group or a divalent non-fluorinated aryl group.

The fluorinated organic group is a divalent organic group having one or more fluorine atoms. From the perspective that it is possible to obtain a crosslinked product that exhibits elastomeric characteristics at a much higher temperature and furthermore in a much wider temperature range, and to obtain a crosslinked product that has much better heat resistance and that adheres much more firmly to a substrate, $Rf^2$ is preferably a linear or branched chain fluorinated alkylene group.

From the perspective that it is possible to obtain a crosslinked product that exhibits elastomeric characteristics at a much higher temperature and furthermore in a much wider temperature range, and to obtain a crosslinked product that has much better heat resistance and that adheres much more firmly to a substrate, the fluorinated alkylene group is preferably a perfluoroalkylene group.

From the perspective that it is possible to obtain a crosslinked product that exhibits elastomeric characteristics at a much higher temperature and furthermore in a much wider temperature range, and to obtain a crosslinked product that has much better heat resistance and that adheres much more firmly to a substrate, the number of carbon atoms of the fluorinated alkylene group is preferably 1 to 15, and more preferably 2 or more, and more preferably 10 or less, and further preferably 5 or less.

From the perspective that it is possible to obtain a crosslinked product that exhibits elastomeric characteristics at a much higher temperature and furthermore in a much wider temperature range, and to obtain a crosslinked product that has much better heat resistance and that adheres much more firmly to a substrate, the fluorinated alkylene group is preferably $-C(CF_3)_2-$.

Each $R^1$ is independently OH, an optionally-substituted linear or branched chain alkoxy group, an optionally-substituted aromatic oxy group, or a halogen atom.

The number of carbon atoms of the alkoxy group as $R^1$ is preferably 1 to 12, and more preferably 1 to 6.

The optional substituent of the alkoxy group and the aromatic oxy group as $R^1$ is preferably an alkoxy group, an alkyl group, a fluorinated alkyl group, a halo group (halogen atom), a nitro group, a cyano group, or an ester group, and more preferably is an alkoxy group.

Examples of the aromatic oxy group as $R^1$ include a phenoxy group that does not have a substituent, an optionally-substituted triazinyloxy group, and the like.

It is preferable that each $R^1$ is independently OH, a phenoxy group that does not have a substituent, a methoxy group, an ethoxy group, a chlorine atom, or the following.

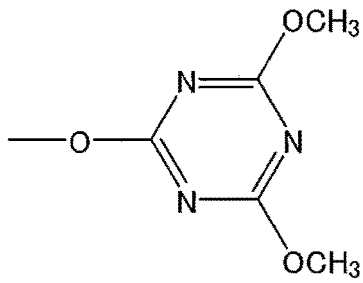

Each $R^2$ is independently H or a monovalent organic group. The monovalent organic group is a monovalent group containing a carbon atom or a group formed by removing one hydrogen atom from an organic compound. Examples of the monovalent organic group include an optionally-substituted aliphatic hydrocarbon group and an optionally-substituted aromatic group.
Specific examples of the monovalent organic group include a lower alkyl group having 1 to 10 carbon atoms, and particularly 1 to 6 carbon atoms, such as $-CH_3$, $-C_2H_5$, and $-C_3H_7$; a fluorine atom-containing lower alkyl group having 1 to 10 carbon atoms, and particularly 1 to 6 carbon atoms, such as $-CF_3$, $-C_2F_5$, $-CH_2F$, $-CH_2CF_3$, and $-CH_2C_2F_5$; a phenyl group (without a substituent); a benzyl group (without a substituent); a phenyl group or a benzyl group in which 1 to 5 hydrogen atoms are substituted with a fluorine atom, such as $-C_6F_5$ and $-CH_2C_6F_5$; and a phenyl group or a benzyl group in which 1 to 5 hydrogen atoms are substituted with a $-CF_3$, such as $-C_6H_{5-n}(CF_3)_n$, $-CH_2C_6H_{5-n}(CF_3)_n$ (n is an integer of 1 to 5) .
It is preferable that each $R^2$ is independently H or an optionally-substituted aromatic group, more preferably H or an optionally-substituted phenyl group, and further preferably H, a phenyl group not having a substituent, or a phenyl group substituted with a fluorine atom-containing alkyl group having 1 to 10 carbon atoms.
From the perspective that it is possible to obtain a crosslinked product that exhibits elastomeric characteristics at a much higher temperature and furthermore in a much wider temperature range, and to obtain a crosslinked product that has much better heat resistance and that adheres much more firmly to a substrate, $R^2$ is preferably a phenyl group not having a substituent, or a phenyl group substituted with a fluorine atom-containing alkyl group having 1 to 10 carbon atoms.
The amide compound of the present disclosure has a crosslinking site at a molecular end in addition to having the above-mentioned repeating unit. Therefore, the amide compound of the present disclosure has crosslinkability, and a crosslinked product can be obtained by crosslinking the amide compound of the present disclosure. Since the amide compound of the present disclosure has such a structure, the crosslinked product obtained by crosslinking the amide compound of the present disclosure exhibits elastomeric characteristics at a high temperature, and furthermore in a wide temperature range. Further, since the amide compound of the present disclosure has such a structure, the crosslinked product obtained by crosslinking the amide compound of the present disclosure has excellent heat resistance and firmly adheres to a substrate.
The amide compound of the present disclosure has a crosslinking site at a molecular end. The molecular end is the end of the chain including the repeating unit represented by Formula (1). Therefore, there are usually two molecular ends. The amide compound of the present disclosure can have a crosslinking site at one or both of the two ends of the main chain of the molecular end, and from the viewpoint of the elastomeric characteristics, heat resistance and adhesiveness of the obtained crosslinked product, it is preferable to have a crosslinking site at both of the two ends of the main chain. Further, it is preferable that the amide compound of the present disclosure has a crosslinking site at all of the molecular ends of the compound.
The crosslinking site of the amide compound of the present disclosure is represented by Formula (2).

Formula (2): $-C{\equiv}CX^1$

(In Formula (2), $X^1$ represents H or a monovalent organic group.)
$X^1$ is H or a monovalent organic group. The monovalent organic group is a monovalent group containing a carbon atom or a group formed by removing one hydrogen atom from an organic compound. Examples of the monovalent organic group include an optionally-substituted aliphatic hydrocarbon group and an optionally-substituted aromatic group.
Specific examples of the monovalent organic group include a lower alkyl group having 1 to 10 carbon atoms, and particularly 1 to 6 carbon atoms, such as $-CH_3$, $-C_2H_5$, and $-C_3H_7$; a fluorine atom-containing lower alkyl group having 1 to 10 carbon atoms, and particularly 1 to 6 carbon atoms, such as $-CF_3$, $-C_2F_5$, $-CH_2F$, $-CH_2CF_3$, and $-CH_2C_2F_5$; a phenyl group (without a substituent); a benzyl group (without a substituent); a phenyl group or a benzyl group in which 1 to 5 hydrogen atoms are substituted with a fluorine atom, such as $-C_6F_5$ and $-CH_2C_6F_5$; and a phenyl group or a benzyl group in which 1 to 5 hydrogen atoms are substituted with a $-CF_3$, such as $-C_6H_{5-n}(CF_3)_n$, $-CH_2C_6H_{5-n}(CF_3)_n$ (n is an integer of 1 to 5) .
It is preferable that $X^1$ is independently H or an optionally-substituted aromatic group, more preferably H or an optionally-substituted phenyl group, and further preferably H, a phenyl group not having a substituent, or a phenyl group substituted with a fluorine atom-containing alkyl group having 1 to 10 carbon atoms.
From the perspective that it is possible to obtain a crosslinked product that exhibits elastomeric characteristics at a much higher temperature and furthermore in a much wider temperature range, and to obtain a crosslinked product that has much better heat resistance and that adheres much more firmly to a substrate, $X^1$ is preferably a phenyl group not having a substituent, or a phenyl group substituted with a fluorine atom-containing alkyl group having 1 to 10 carbon atoms, and more preferably a phenyl group not having a substituent.
The crosslinking site represented by Formula (2) can be bonded to a chain including the repeating unit represented by Formula (1) via an amide bond. Examples of the amide compound of the present disclosure include compounds represented by any of the following formulas.

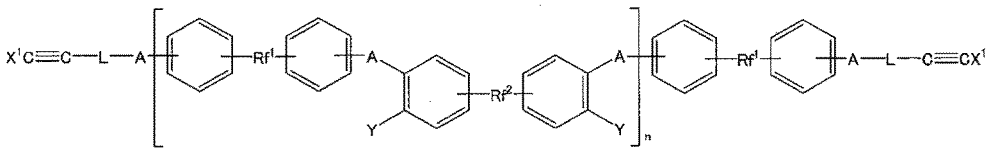

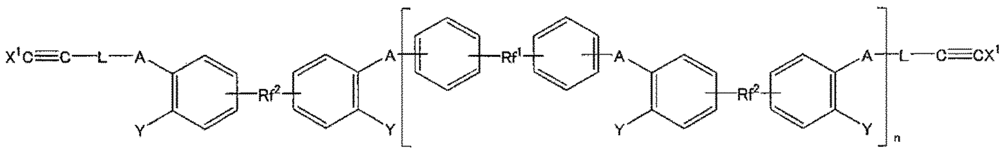

(In the formulas, $Rf^1$, $Rf^2$, A, and Y are the same as in Formula (1) . $X^1$ is the same as in Formula (2). L represents a linking group, and n represents the average degree of polymerization of the repeating unit represented by Formula (1).)

Examples of the linking group L include a single bond, an optionally-substituted alkylene group, an optionally-substituted arylene group, and the like. Of these, an optionally-substituted arylene group is preferable. The substituent is preferably $-COR^1$ or $-NHR^2$ ($R^1$ and $R^2$ are as described above) .

A structure including such a crosslinking site can be formed by modifying an end of the chain including the repeating unit represented by Formula (1) with a compound having the crosslinking site represented by Formula (2) (for example, compound (6) described later).

The repeating unit represented by Formula (1) is preferably a repeating unit represented by Formula (1a).

Formula (1a):

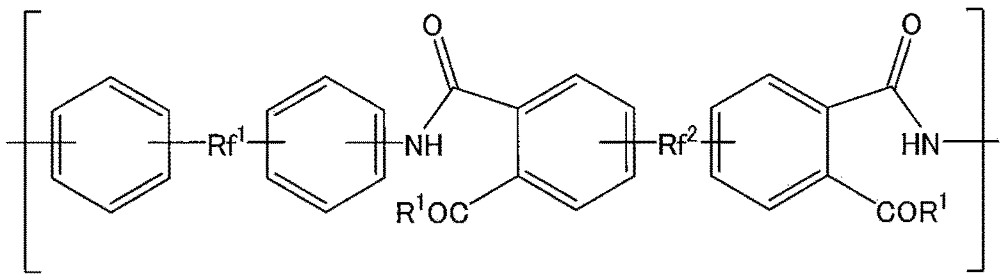

(In Formula (1a), $Rf^1$, $Rf^2$, and $R^1$ are the same as in Formula (1).)

Examples of the amide compound containing the repeating unit represented by Formula (1a) include a compound represented by the following formula.

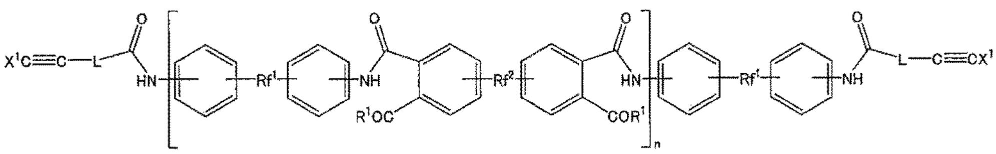

(In the formula, $Rf^1$, $Rf^2$, and $R^1$ are the same as in Formula (1). $X^1$ is the same as in Formula (2). L represents a linking group, and n represents the average degree of polymerization of the repeating unit represented by Formula (1a).)

For the repeating unit represented by Formula (1), the amide compound containing the repeating unit represented by Formula (1a) can be suitably used as a precursor of the nitrogen-containing heterocyclic ring-containing compound (imide compound) having the repeating unit represented by Formula (3a). Further, by thermally crosslinking the amide compound containing the repeating unit represented by Formula (1a), the crosslinked product described later can be obtained, and the obtained crosslinked product exhibits elastomeric characteristics at a high temperature and furthermore in a wide temperature range, while also having excellent heat resistance and firmly adhering to a substrate.

$Rf^1$, $Rf^2$, and $R^1$ in Formula (1a) are the same as in Formula (1). By having the same suitable structure as Formula (1), a crosslinked product can be obtained that exhibits elastomeric characteristics at a much higher temperature and furthermore in a much wider temperature range, and a crosslinked product can be obtained that has much better

heat resistance and that adheres much more firmly to a substrate.

Further, the repeating unit represented by Formula (1) is preferably a repeating unit represented by Formula (1b).

Formula (1b):

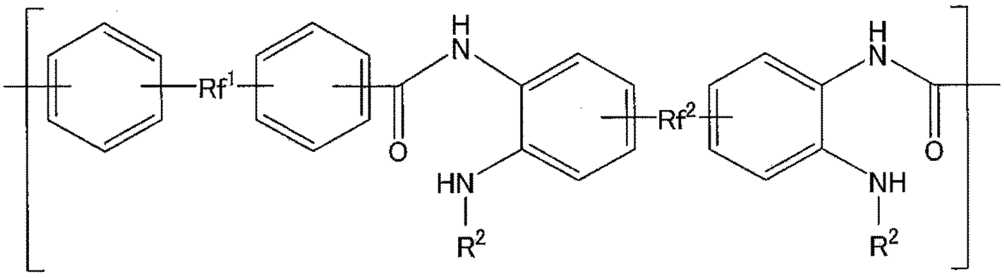

(In Formula (1b), $Rf^1$, $Rf^2$, and $R^2$ are the same as in Formula (1) .)

Examples of the amide compound containing the repeating unit represented by Formula (1b) include a compound represented by the following formula.

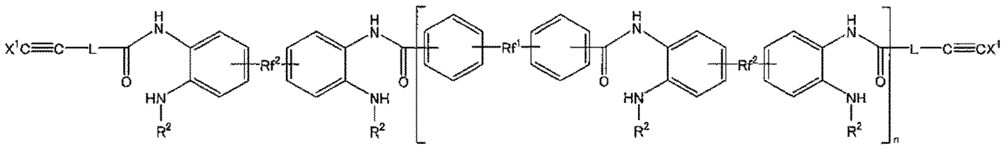

(In the formula, $Rf^1$, $Rf^2$, and $R^2$ are the same as in Formula (1). $X^1$ is the same as in Formula (2). L represents a linking group, and n represents the average degree of polymerization of the repeating unit represented by Formula (1b).)

For the repeating unit represented by Formula (1), the amide compound containing the repeating unit represented by Formula (1b) can be suitably used as a precursor of the nitrogen-containing heterocyclic ring-containing compound (benzimidazole compound) having the repeating unit represented by Formula (3b). Further, by thermally crosslinking the amide compound containing the repeating unit represented by Formula (1b), the crosslinked product described later can be obtained, and the obtained crosslinked product exhibits elastomeric characteristics at a high temperature and furthermore in a wide temperature range, while also having excellent heat resistance and firmly adhering to a substrate.

$Rf^1$, $Rf^2$, and $R^2$ in Formula (1b) are the same as in Formula (1). By having the same suitable structure as Formula (1), a crosslinked product can be obtained that exhibits elastomeric characteristics at a much higher temperature and furthermore in a much wider temperature range, and a crosslinked product can be obtained that has much better heat resistance and that adheres much more firmly to a substrate.

The glass transition temperature of the amide compound of the present disclosure is preferably 100°C to 300°C, and more preferably 100°C to 250°C. The glass transition temperature is a value measured by differential scanning calorimetry (DSC).

In the amide compound of the present disclosure, the average degree of polymerization of the repeating unit represented by Formula (1) is preferably 10 or less, more preferably 6 or less, and further preferably 4 or less, and may be 2 or more, or may be 3 or more. The average degree of polymerization is determined from the molar ratio of the monomers and the number average molecular weight of the amide compound when synthesizing the amide compound of the present disclosure.

From the perspective that excellent characteristics can be obtained after crosslinking, the amide compound is preferably an oligomer having a relatively small average degree of polymerization. For example, a polymer having an average degree of polymerization of more than 10 may be used.

The number average molecular weight (Mn) of the amide compound of the present disclosure is, in terms of standard polystyrene by gel permeation chromatography, preferably 1,000 or more, and more preferably 3,000 or more, and preferably 10,000 or less, and more preferably 5,000 or less.

The molecular weight distribution (Mw/Mn) of the amide compound of the present disclosure is, in terms of standard polystyrene by gel permeation chromatography, preferably 1.2 or more, and more preferably 2 or more, and preferably 4 or less.

The logarithmic viscosity $\eta_{inh}$ (dL/g) of the amide compound of the present disclosure is preferably 0.1 dL/g or more, and preferably 0.5 dL/g or less. The logarithmic viscosity $\eta_{inh}$ (dL/g) can be calculated by the following formula

by dissolving the amide compound in N-methyl-2-pyrrolidone (NMP) as a solvent to prepare a solution having a solution concentration of 0.5 g/dL and measuring the solution viscosity of the prepared solution at 30°C.

$$\text{Logarithmic viscosity } \eta_{inh} = \ln(\text{solution viscosity / solvent viscosity}) / \text{solution concentration}$$

The amide compound of the present disclosure can be suitably used as a precursor of the nitrogen-containing heterocyclic ring-containing compound having a repeating unit represented by Formula (3) described below. Further, by thermally crosslinking the amide compound of the present disclosure, the crosslinked product described later can be obtained.

<Nitrogen-containing heterocyclic ring-containing compound>

The nitrogen-containing heterocyclic ring-containing compound of the present disclosure includes a repeating unit represented by Formula (3) and a crosslinking site represented by Formula (2) at a molecular end.

Formula (3):

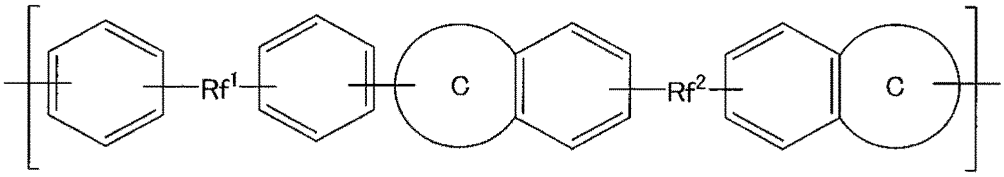

(In Formula (3), $Rf^1$ and $Rf^2$ are the same as in Formula (1), and ring C represents an optionally-substituted imide ring or benzimidazole ring.)

Formula (2): $-C\equiv CX^1$

(In Formula (2), $X^1$ is as described above.)

$Rf^1$ and $Rf^2$ are the same as in Formula (1). By making $Rf^1$ and $Rf^2$ have the same suitable structure as in Formula (1), a crosslinked product that exhibits elastomeric characteristics at a much higher temperature and furthermore in a much wider temperature range, and a crosslinked product that has much better heat resistance and that adheres much more firmly to a substrate, can be obtained.

The ring C is an optionally-substituted imide ring or benzimidazole ring. It is preferable that the imide ring does not have a substituent. The benzimidazole ring is optionally substituted. Examples of the substituent that benzimidazole can have include a monovalent organic group, and the groups described for $R^2$ of Formula (1) are preferable.

The nitrogen-containing heterocyclic ring-containing compound of the present disclosure has a crosslinking site at a molecular end in addition to having the above-mentioned repeating unit. Therefore, the nitrogen-containing heterocyclic ring-containing compound of the present disclosure has crosslinkability, and a crosslinked product can be obtained by crosslinking the nitrogen-containing heterocyclic ring-containing compound of the present disclosure. Since the nitrogen-containing heterocyclic ring-containing compound of the present disclosure has such a structure, the crosslinked product obtained by crosslinking the nitrogen-containing heterocyclic ring-containing compound of the present disclosure exhibits elastomeric characteristics at a high temperature, and furthermore in a wide temperature range. Further, since the nitrogen-containing heterocyclic ring-containing compound of the present disclosure has such a structure, the crosslinked product obtained by crosslinking the nitrogen-containing heterocyclic ring-containing compound of the present disclosure has excellent heat resistance and firmly adheres to a substrate.

The nitrogen-containing heterocyclic ring-containing compound of the present disclosure has a crosslinking site at a molecular end. The molecular end is the end of the chain including the repeating unit represented by Formula (3). Therefore, there are usually two molecular ends. The nitrogen-containing heterocyclic ring-containing compound of the present disclosure can have a crosslinking site at one or both of the two ends of the main chain of the molecular end, and from the viewpoint of the elastomeric characteristics, heat resistance and adhesiveness of the obtained crosslinked product, it is preferable to have a crosslinking site at both of the two ends of the main chain. Further, it is preferable that the nitrogen-containing heterocyclic ring-containing compound of the present disclosure has a crosslinking site at all of the molecular ends of the compound.

The crosslinking site of the nitrogen-containing heterocyclic ring-containing compound of the present disclosure is represented by Formula (2). The crosslinking site represented by Formula (2) of the nitrogen-containing heterocyclic ring-containing compound of the present disclosure is the same as the crosslinking site of the amide compound of the present disclosure. By making the crosslinking site represented by Formula (2) included in the nitrogen-containing heterocyclic ring-containing compound of the present disclosure have the same suitable structure as that of the amide compound of the present disclosure, a crosslinked product that exhibits elastomeric characteristics at a much higher temperature and furthermore in a much wider temperature range, and a crosslinked product that has much better heat resistance and that adheres much more firmly to a substrate, can be obtained.

The crosslinking site represented by Formula (2) can be bonded to a chain including the repeating unit represented by Formula (3) via an imide ring. Examples of the nitrogen-containing heterocyclic ring-containing compound of the present disclosure include compounds represented by any of the following formulas.

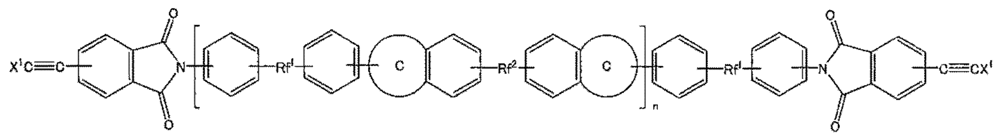

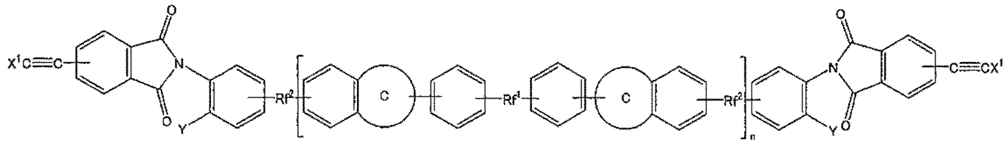

(In the formulas, $Rf^1$, $Rf^2$, and Y are the same as in Formula (1). The ring C is the same as in Formula (3), $X^1$ is the same as in Formula (2), and n represents the average degree of polymerization of the repeating unit represented by Formula (3).)

A structure including such a crosslinking site can be formed by modifying an end of the chain including the repeating unit represented by Formula (3) with a compound having the crosslinking site represented by Formula (2) (for example, compound (6) described later).

The repeating unit represented by Formula (3) is preferably a repeating unit represented by Formula (3a). Formula (3a):

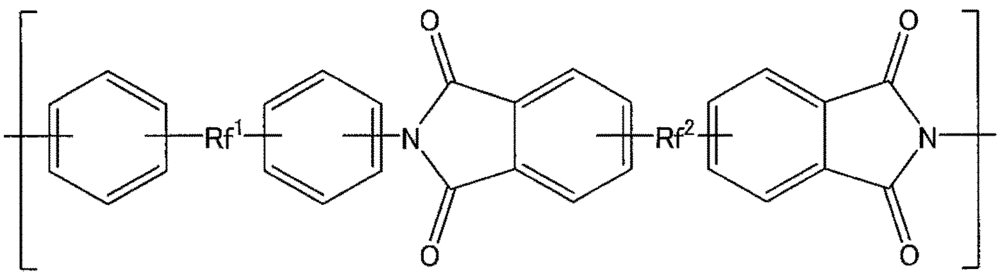

(In Formula (3a), $Rf^1$ and $Rf^2$ are the same as in Formula (1).)

Examples of the nitrogen-containing heterocyclic ring-containing compound containing the repeating unit represented by Formula (3a) include a compound represented by the following formula.

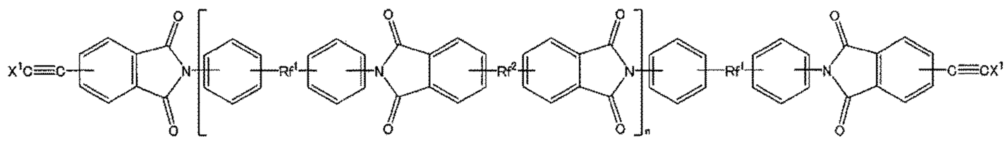

(In the formula, $Rf^1$ and $Rf^2$ are the same as in Formula (1). $X^1$ is the same as in Formula (2), and n represents the average degree of polymerization of the repeating unit represented by Formula (3a).)

By crosslinking the nitrogen-containing heterocyclic ring-containing compound (imide compound) containing the repeating unit represented by Formula (3a) as the repeating unit represented by Formula (3), the crosslinked product described later can be obtained, and the obtained crosslinked product exhibits elastomeric characteristics at a high

temperature and furthermore in a wide temperature range, while also having excellent heat resistance and firmly adhering to a substrate.

$Rf^1$ and $Rf^2$ in Formula (3a) are the same as in Formula (1). By having the same suitable structure as Formula (1), a crosslinked product can be obtained that exhibits elastomeric characteristics at a much higher temperature and furthermore in a much wider temperature range, and a crosslinked product can be obtained that has much better heat resistance and that adheres much more firmly to a substrate.

Further, the repeating unit represented by Formula (3) is preferably a repeating unit represented by Formula (3b).

Formula (3b):

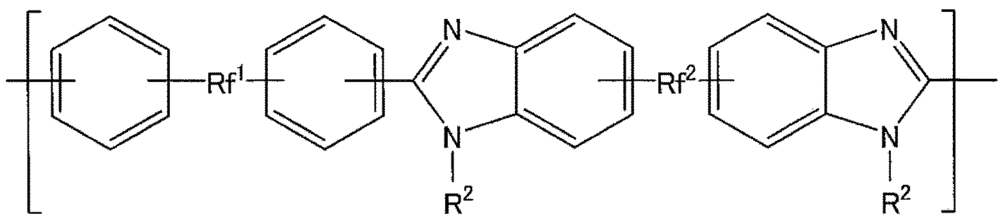

(In Formula (3b), $Rf^1$, $Rf^2$, and $R^2$ are the same as in Formula (1) .)

Examples of the nitrogen-containing heterocyclic ring-containing compound containing the repeating unit represented by Formula (3b) include a compound represented by the following formula.

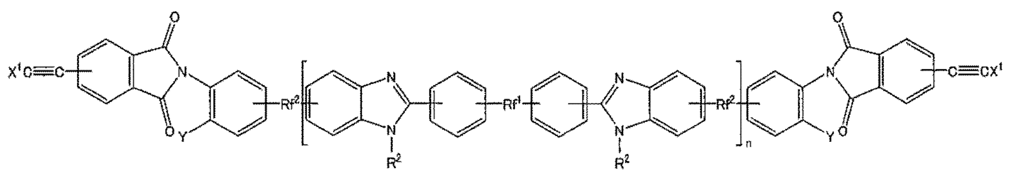

(In the formula, $Rf^1$, $Rf^2$, $R^2$, and Y are the same as in Formula (1). $X^1$ is the same as in Formula (2), and n represents the average degree of polymerization of the repeating unit represented by Formula (3b).)

By crosslinking the nitrogen-containing heterocyclic ring-containing compound (benzimidazole compound) containing the repeating unit represented by Formula (3b) as the repeating unit represented by Formula (3), the crosslinked product described later can be obtained, and the obtained crosslinked product exhibits elastomeric characteristics at a high temperature and furthermore in a wide temperature range, while also having excellent heat resistance and firmly adhering to a substrate.

$Rf^1$, $Rf^2$, and $R^2$ in Formula (3b) are the same as in Formula (1). By having the same suitable structure as Formula (1), a crosslinked product can be obtained that exhibits elastomeric characteristics at a much higher temperature and furthermore in a much wider temperature range, and a crosslinked product can be obtained that has much better heat resistance and that adheres much more firmly to a substrate.

From the perspective that it is possible to obtain a nitrogen-containing heterocyclic ring-containing compound that exhibits much better heat resistance and adheres much more firmly to a substrate, the repeating unit represented by Formula (3b) is preferably a repeating unit represented by Formula (3b-1) or Formula (3b-2).

Formula (3b-1)

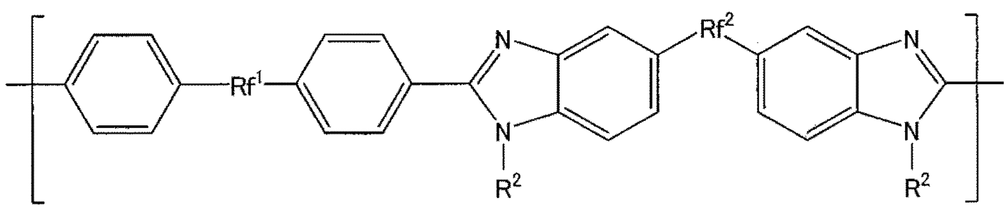

(In Formula (3b-1), $Rf^1$, $Rf^2$, and $R^2$ are the same as in Formula (1).)

Formula (3b-2)

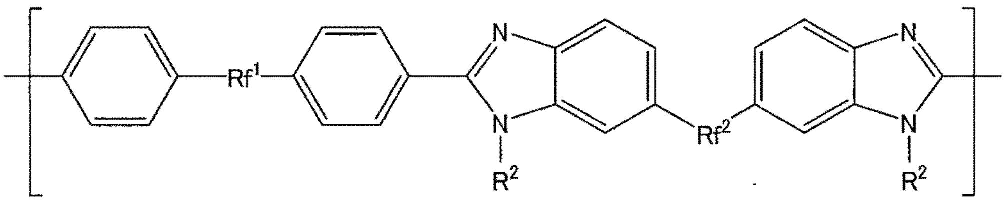

(In Formula (3b-2), $Rf^1$, $Rf^2$, and $R^2$ are the same as in Formula (1) .)

The glass transition temperature of the nitrogen-containing heterocyclic ring-containing compound of the present disclosure is preferably 80°C to 250°C, and more preferably 100°C to 200°C. The glass transition temperature is a value measured by differential scanning calorimetry (DSC).

In the nitrogen-containing heterocyclic ring-containing compound of the present disclosure, the average degree of polymerization of the repeating unit represented by Formula (3) is preferably 10 or less, more preferably 6 or less, and further preferably 4 or less, and may be 2 or more, or may be 3 or more. The average degree of polymerization is determined from the added molar ratio and the number average molecular weight of the nitrogen-containing heterocyclic ring-containing compound of the present disclosure.

From the perspective that excellent characteristics can be obtained after crosslinking, the nitrogen-containing heterocyclic ring-containing compound is preferably an oligomer having a relatively small average degree of polymerization. For example, a polymer having an average degree of polymerization of more than 10 may be used.

The number average molecular weight (Mn) of the nitrogen-containing heterocyclic ring-containing compound of the present disclosure is, in terms of standard polystyrene by gel permeation chromatography, preferably 1,000 or more, and more preferably 3,000 or more, and preferably 10,000 or less, and more preferably 5,000 or less.

The molecular weight distribution (Mw/Mn) of the nitrogen-containing heterocyclic ring-containing compound of the present disclosure is, in terms of standard polystyrene by gel permeation chromatography, preferably 1.2 or more, and more preferably 2 or more, and preferably 5 or less, and more preferably 4 or less.

The logarithmic viscosity $\eta_{inh}$ (dL/g) of the nitrogen-containing heterocyclic ring-containing compound of the present disclosure is preferably 0.1 dL/g or more, and preferably 0.5 dL/g or less. The logarithmic viscosity $\eta_{inh}$ (dL/g) can be calculated by the following formula by dissolving the nitrogen-containing heterocyclic ring-containing compound in N-methyl-2-pyrrolidone (NMP) as a solvent to prepare a solution having a solution concentration of 0.5 g/dL and measuring the solution viscosity of the prepared solution at 30°C.

$$\text{Logarithmic viscosity } \eta_{inh} = \ln(\text{solution viscosity / solvent viscosity}) / \text{solution concentration}$$

The crosslinked product described later can be obtained by crosslinking the nitrogen-containing heterocyclic ring-containing compound of the present disclosure.

<Crosslinked product>

The crosslinked product of the present disclosure can be obtained by crosslinking the amide compound or the nitrogen-containing heterocyclic ring-containing compound. For example, it is presumed that by heating the amide compound or the nitrogen-containing heterocyclic ring-containing compound, three crosslinking sites located at the end of each of three molecules react with each other to form a ring, thereby forming a crosslinked product. Due to the skeleton formed by the repeating unit of the amide compound or nitrogen-containing heterocyclic ring-containing compound, and the crosslinked structure formed by crosslinking, the crosslinked product of the present disclosure exhibits elastomeric characteristics at a high temperature, and furthermore, in a wide temperature range, and has excellent heat resistance and adheres firmly to a substrate.

As used herein, "elastomeric characteristics" (rubber characteristics) refers to characteristics that enable the crosslinked product to stretch and to retain its original length when the force required to stretch the crosslinked product is no longer applied. The elastomeric characteristics can be confirmed by dynamic viscoelasticity analysis (DMA) of the crosslinked product to obtain a DMA curve and evaluating the temperature dependence of the storage elastic modulus (E').

The crosslinked product of the present disclosure may contain a filler. Examples of the filler include imide-based fillers having an imide structure such as polyimide, polyamideimide, and polyetherimide, organic fillers made of

an engineering plastic such as polyarylate, polysulfone, polyethersulfone, polyphenylene sulfide, polyether ether ketone, polyether ketone, and polyoxybenzoate, metal oxide fillers such as aluminum oxide, silicon oxide, and yttrium oxide, metal carbides such as silicon carbide and aluminum carbide, metal nitride fillers such as silicon nitride and aluminum nitride, and inorganic fillers such as aluminum fluoride, carbon fluoride, barium sulfate, carbon black, silica, clay, and talc.

Among these, from the viewpoint of a shielding effect of various plasmas, carbon black, aluminum oxide, yttrium oxide, silicon oxide, polyimide, and carbon fluoride are preferable.

Further, the above-mentioned inorganic fillers and organic fillers may be blended alone or in combination of two or more.

The content of the filler in the crosslinked product is, based on 100 parts by mass of the total of the amide compound and the nitrogen-containing heterocyclic ring-containing compound, preferably 0.5 to 100 parts by mass, more preferably 5 to 50 parts by mass.

Further, the crosslinked product of the present disclosure may optionally contain ordinary additives that are blended in rubber, for example, a processing aid, a plasticizer, a coloring agent, and the like.

<Method for producing amide compound>

The amide compound of the present disclosure can be suitably produced by polymerizing a compound (4) represented by Formula (4) and a compound (5) represented by Formula (5) to obtain a compound having the repeating unit represented by Formula (1), and then reacting the group Y of the molecular end of the obtained compound with a compound (6) represented by Formula (6) to introduce the crosslinking site represented by Formula (2) onto the molecular end.

Formula (4):

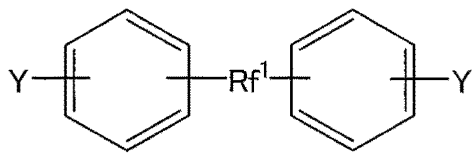

(In Formula (4), $Rf^1$ and Y are the same as in Formula (1).)

Formula (5):

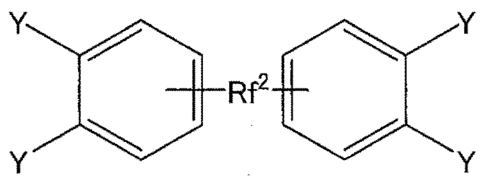

(In Formula (5), $Rf^2$ and Y are the same as in Formula (1). However, when Y in Formula (4) is $-COR^1$, Y in Formula (5) is $-NHR^2$. Further, when Y in Formula (4) is $-NHR^2$, Y in Formula (5) is $-COR^1$. Further, when Y in Formula (5) is $-COR^1$, two adjacent Y may be bonded to each other via an acid anhydride bond (-Co-O-Co-) to form a ring together with the two carbon atoms that the two Y's are bonded to. $R^1$ and $R^2$ are the same as in Formula (1).)

Formula (6) : $R^3-C\equiv CX^1$

(In Formula (6), $R^3$ is a monovalent organic group having a reactive group capable of reacting with the group Y of the molecular end of the compound obtained by polymerizing the compound (4) and the compound (5), and $X^1$ is the same as in Formula (2).)

As the compound (4) and the compound (5), the compound (4) is preferably a compound (4a) represented by general formula (4a) and the compound (5) is preferably a compound (5a) represented by general formula (5a), or the compound (4) is preferably a compound (4b) represented by general formula (4b) and the compound (5) is preferably a compound (5b) represented by general formula (5b). The amide compound having a repeating unit represented by Formula (1a) can be obtained by polymerizing the compound (4a) and the compound (5a). Further, the amide compound having a repeating unit represented by Formula (1b) can be obtained by polymerizing the compound (4b) and the compound (5b).

General formula (4a):

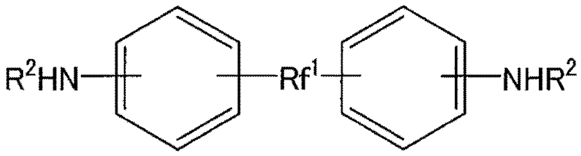

(In Formula (4a), $Rf^1$ and $R^2$ are the same as in Formula (1).)

General formula (5a):

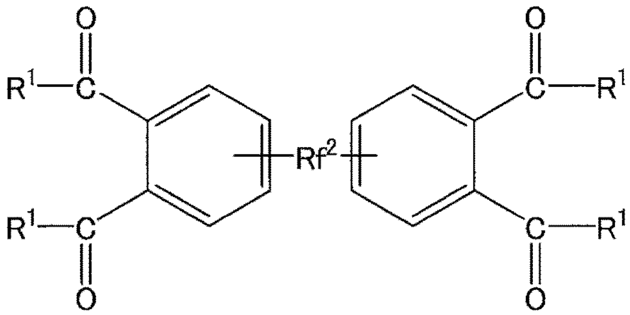

(In Formula (5a), $Rf^2$ and $R^1$ are the same as in Formula (1).)

General formula (4b):

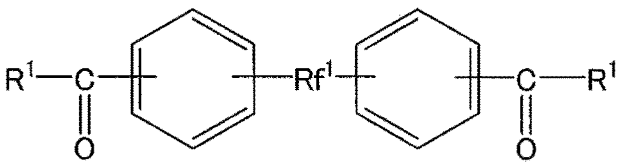

(In Formula (4b), $Rf^1$ and $R^1$ are the same as in Formula (1).)

General formula (5b):

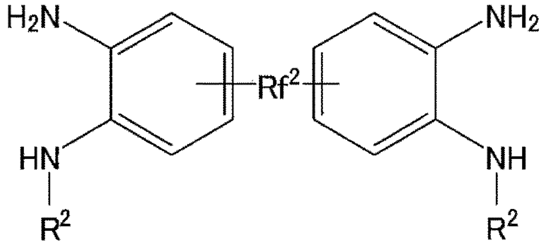

(In Formula (5b), $Rf^2$ and $R^2$ are the same as in Formula (1).)

The polymerization of the compound (4) and the compound (5) can be carried out in a solvent. It is desirable that the solvent does not substantially react with the compound (4) and the compound (5), has the property of dissolving the compound (4) and the compound (5) well, and is a good solvent for the compound obtained by polymerizing the compound (4) and the compound (5). Such a solvent is not limited, and examples thereof include dimethyl sulfoxide (DMSO), N,N-dimethylformamide (DMF), N,N-dimethylacetamide (DMAc), N-methyl-2-pyrrolidone (NMP), 1,3-dimethylimidazolidone (DMI), sulfolane, tetrahydrofuran (THF), and acetone. Of these, N-methyl-2-pyrrolidone (NMP) and 1,3-dimethylimidazolidone (DMI) are preferable. The amount of these solvents used is, based on 0.1 mol of the compound (4) or the compound (5), usually 10 to 1,000 mL, preferably 50 to 400 mL.

The polymerization can also be carried out in the presence of additives. For example, an inorganic salt such

as lithium chloride or calcium chloride may be added in order to obtain a compound having a large molecular weight. OF these, lithium chloride is preferable as the additive. The amount of the additive added is, based on the amount of the solvent, preferably 10% by mass or less, and more preferably 5% by mass or less.

The polymerization can be carried out by, for example, dissolving any one of the compound (4) and the compound (5) in the solvent, adding the other compound to the resulting solution, and then carrying out the reaction while stirring under an inert atmosphere such as nitrogen. The polymerization temperature is preferably -50 to 150°C, more preferably 0 to 100°C, and further preferably 35 to 80°C. The polymerization time is preferably 0.1 to 50 hours, and more preferably 1 to 24 hours.

The average degree of polymerization of the repeating unit represented by Formula (1) can be adjusted by changing the molar ratio of the compound (4) to the compound (5), the concentration of the polymerization solution, the polymerization temperature, the polymerization time, and the like.

After completion of the polymerization, a highly pure compound may be obtained by charging the reaction mixture into a poor solvent such as methanol or water to separate the polymer, and then purifying by a reprecipitation method to remove by-products, inorganic salts, and the like.

Next, the obtained compound is reacted with the compound represented by Formula (6) to introduce the crosslinking site represented by Formula (2) at a molecular end of the compound. The compound represented by Formula (6) has a monovalent organic group $R^3$ having a reactive group capable of reacting with the group Y of the molecular end of the obtained compound. The reactive group in the organic group $R^3$ reacts with the group Y of the molecular end of the obtained compound, and the compound represented by Formula (6) is added to the compound obtained by polymerizing the compound (4) and the compound (5).

Examples of the reactive group of $R^3$ in Formula (6) include a hydroxyl group, a carboxyl group, an acid anhydride group, and an amino group. When the group Y of the molecular end of the obtained compound is $-COR^1$, the reactive group of $R^3$ in Formula (6) is preferably $-NHR^2$. Further, when the group Y of the molecular end of the obtained compound is $-NHR^2$, the reactive group of $R^3$ in Formula (6) is preferably $-COR^1$ or an acid anhydride group (-CO-O-CO-) . $R^1$ and $R^2$ are the same as in Formula (1) .

Examples of the compound (6) include 4-phenylethynylphthalic anhydride (PEPA), propargyl alcohol, phenylpropargyl alcohol, propargylamine, phenylethynylaniline, ethynylaniline, and the like.

The amount of the compound (6) used may be adjusted according to the equivalent amount of the group Y of the molecular end of the compound obtained by polymerizing the compound (4) and the compound (5). For example, the amount of compound (6) used may be in the range of 2.0 to 2.5 mol based on 1 mol of the compound obtained by polymerizing the compound (4) and the compound (5) .

The reaction conditions between the compound obtained by polymerizing the compound (4) and the compound (5) and the compound (6) are not limited. The reaction temperature is preferably 0 to 100°C, and more preferably 20 to 80°C. The reaction time is preferably 0.1 to 50 hours, and more preferably 1 to 24 hours.

The reaction can be carried out in a solvent. It is preferable that the solvent can dissolve the compound obtained by the polymerization of the compound (4) and the compound (5). Such a solvent is not limited, and examples thereof include dimethyl sulfoxide (DMSO), N,N-dimethylformamide (DMF), N,N-dimethylacetamide (DMAc), N-methyl-2-pyrrolidone (NMP), 1,3-dimethylimidazolidone (DMI), sulfolane, tetrahydrofuran (THF), and acetone. Of these, N-methyl-2-pyrrolidone (NMP) and 1,3-dimethylimidazolidone (DMI) are preferable. The amount of these solvents used is, based on 0.1 mol of the compound, usually 10 to 1,000 mL, preferably 50 to 400 mL.

After completion of the reaction, a highly pure amide compound may be obtained by charging the reaction mixture into a poor solvent such as methanol or water to separate the amide compound, and then purifying by a reprecipitation method to remove by-products, inorganic salts, and the like.

Of the compounds (4a), the compound (4a-1) represented by Formula (4a-1) is a novel compound.

Formula (4a-1):

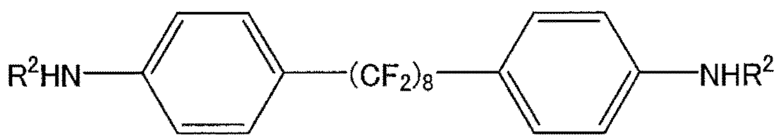

(In Formula (4a-1), $R^2$ is the same as in Formula (1).)

The compound (4a-1) can be produced by reacting the compound (7a) represented by Formula (7a) with 1,8-diiodoperfluorooctane.

Formula (7a):

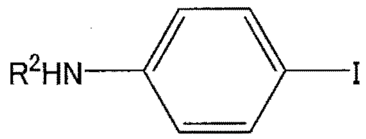

(In Formula (7a), $R^2$ is the same as in Formula (1).)

The reaction between the compound (7a) and 1,8-diiodoperfluorooctane can be carried out in a solvent. It is desirable that the solvent does not substantially react with the compound (7a) and 1,8-diiodoperfluorooctane, has the property of dissolving the compound (7a) and 1,8-diiodoperfluorooctane well, and is a good solvent for the obtained compound (4a-1). Such a solvent is not limited, and examples thereof include dimethyl sulfoxide (DMSO), N,N-dimethylformamide (DMF), N,N-dimethylacetamide (DMAc), N-methyl-2-pyrrolidone (NMP), 1,3-dimethylimidazolidone (DMI), sulfolane, tetrahydrofuran (THF), and acetone. Of these, dimethyl sulfoxide (DMSO) is preferable. The amount of these solvents used is, based on 0.1 mol of the compound (7a) or 1,8-diiodoperfluorooctane, usually 10 to 1,000 mL, preferably 50 to 400 mL.

The reaction of between the compound (7a) and 1,8-diiodoperfluorooctane can be carried out by, for example, dissolving any one of the compound (7a) and 1,8-diiodoperfluorooctane in the solvent, adding the other compound to the resulting solution, and then carrying out the reaction while stirring under an inert atmosphere such as nitrogen. The reaction temperature is preferably 50 to 150°C, and more preferably 100 to 140°C. The reaction time is preferably 1 to 50 hours, and more preferably 1 to 30 hours.

The reaction between the compound (7a) and 1,8-diiodoperfluorooctane may be carried out in the presence of a catalyst such as a copper compound.

After completion of the reaction, a highly pure compound (4a-1) may be obtained by charging the reaction mixture into a poor solvent such as methanol or water to separate the compound (4a-1), and then purifying by a recrystallization method to remove by-products, inorganic salts, and the like.

Next, a method for producing the compound (4b) will be described. A compound (4b) in which $R^1$ is OH can be produced by reacting a compound (7b1) represented by Formula (7b1) with a compound (7b2) represented by Formula (7b2) to obtain a compound (8) represented by Formula (8), and then oxidizing the obtained compound (8).

Formula (7b1):

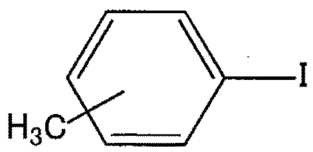

Formula (7b2): I-$Rf^1$-I

(In Formula (7), $Rf^1$ is the same as in Formula (1).)

Formula (8):

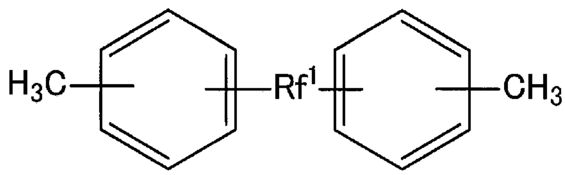

(In Formula (8), $Rf^1$ is the same as in Formula (1).)

The compound (7b1) is more preferably 4-iodotoluene or 3-iodotoluene.

Further, by reacting the compound (4b) in which $R^1$ is OH with a compound (9) represented by Formula (9), a compound (4b) having a desired group represented by $R^1$ (for example, an alkoxy group or an aromatic oxy group) can be produced.

Formula (9): R-OH

(In Formula (9), R is an optionally-substituted linear or branched chain alkyl group or an optionally-substituted aromatic group.)

Further, by reacting the compound (4b) in which $R^1$ is OH with a halogenating agent, a compound (4b) in which $R^1$ is a halogen atom can be produced.

Further, by reacting the compound (4b) in which $R^1$ is OH with a triazine chloride compound, a compound (4b) in which $R^1$ is a triazinyloxy group can be produced.

The reaction between the compound (7b1) and the compound (7b2) can be carried out in a solvent. It is desirable that the solvent does not substantially react with the compound (7b1) and compound (7b2), has the property of dissolving the compound (7b1) and compound (7b2) well, and is a good solvent for the obtained compound (8). Such a solvent is not limited, and examples thereof include dimethyl sulfoxide (DMSO), N,N-dimethylformamide (DMF), N,N-dimethylacetamide (DMAc), N-methyl-2-pyrrolidone (NMP), 1,3-dimethylimidazolidone (DMI), sulfolane, tetrahydrofuran (THF), and acetone. Of these, dimethyl sulfoxide (DMSO) is preferable. The amount of these solvents used is, based on 0.1 mol of the compound (7b1) or compound (7b2), usually 10 to 1,000 mL, preferably 50 to 400 mL.

The reaction between the compound (7b1) and the compound (7b2) can be carried out by, for example, dissolving any one of the compound (7b1) and compound (7b2) in the solvent, adding the other compound to the resulting solution, and then carrying out the reaction while stirring under an inert atmosphere such as nitrogen. The reaction temperature is preferably 50 to 150°C, and more preferably 100 to 140°C. The reaction time is preferably 1 to 50 hours, and more preferably 1 to 30 hours.

The reaction between the compound (7b1) and the compound (7b2) may be carried out in the presence of a catalyst such as copper or a copper compound.

The oxidation of the compound (8) can be performed using, for example, an oxidizing agent such as $CrO_3$ or $KMnO_4$. Further, the oxidation of the compound (8) is preferably carried out in the presence of an acidic compound such as sulfuric acid or acetic acid.

Examples of the halogenating agent used in the reaction between the compound (4b) in which $R^1$ is OH and the halogenating agent include thionyl chloride, phosphorus trichloride, phosphorus pentachloride, and the like. The reaction temperature may be, for example, 20 to 100°C. The reaction between the compound (4b) and the halogenating agent can also be carried out in a solvent. Examples of the solvent include ethers such as diethyl ether and tetrahydrofuran.

The reaction between the compound (4b) in which $R^1$ is OH and the triazine chloride compound can be carried out in a solvent in the presence of N-methylmorpholine (NMM). As a result of this reaction, a triazine-based active diester can be synthesized. It is desirable that the solvent does not substantially react with the compound (4b), the triazine chloride compound, and N-methylmorpholine (NMM), has the property of dissolving the compound (4b), the triazine chloride compound, and N-methylmorpholine (NMM) well, and is a good solvent for the obtained triazine-based active diester. Such a solvent is not limited, and examples thereof include dimethyl sulfoxide (DMSO), N,N-dimethylformamide (DMF), N,N-dimethylacetamide (DMAc), N-methyl-2-pyrrolidone (NMP), 1,3-dimethylimidazolidone (DMI), sulfolane, tetrahydrofuran (THF), and acetone. Of these, N-methyl-2-pyrrolidone (NMP) is preferable. The amount of these solvents used is, based on 0.1 mol of the compound (4b) or triazine chloride compound, usually 10 to 1,000 mL, preferably 50 to 400 mL.

The reaction between the compound (4b) in which $R^1$ is OH and the triazine chloride compound can be carried out by, for example, dissolving any one of the compound (4b) in which $R^1$ is OH and the triazine chloride compound in the solvent, adding the other compound to the resulting solution, and then carrying out the reaction while stirring under an inert atmosphere such as nitrogen. The reaction temperature is preferably 0 to 150°C, more preferably 50 to 150°C, and further preferably 100 to 140°C. The reaction time is preferably 1 to 50 hours, and more preferably 1 to 30 hours.

<Method for producing nitrogen-containing heterocyclic ring-containing compound>

The nitrogen-containing heterocyclic ring-containing compound of the present disclosure can be suitably produced by a production method of obtaining an amide compound by the production method described above, and then cyclodehydrating the amide compound. Further, when producing the amide compound, in a case in which the reaction between the compound (6) and the compound obtained by polymerizing the compound (4) and the compound (5) is carried out in a heated state, a part or all of the compound undergoes cyclodehydration, which may result the nitrogen-containing heterocyclic ring-containing compound of the present disclosure being obtained as a part or all of the product. That is, the present disclosure also includes mixtures of the amide compound and the nitrogen-containing heterocyclic ring-containing compound.

The cyclodehydration of the amide compound can be carried out by heating the amide compound. The heating temperature for the cyclodehydration is preferably a temperature at which the crosslinking reaction does not proceed.

The heating temperature for the cyclodehydration is preferably 110 to 290°C, and more preferably 150 to 260°C. The heating time is preferably 0.1 to 10 hours, and more preferably 0.5 to 8 hours. The cyclodehydration can be carried out in air, in a nitrogen or argon atmosphere, or under reduced pressure.

Generally, a solution of the amide compound or nitrogen-containing heterocyclic ring-containing compound is obtained by the production method described above. The obtained solution may be used as it is for various purposes, or the obtained solution may be charged into a poor solvent such as methanol or water to separate the nitrogen-containing heterocyclic ring-containing compound, which is then dried to produce a powder of the nitrogen-containing heterocyclic ring-containing compound, and the obtained powder may be used.

<Method for producing crosslinked product>

The crosslinked product of the present disclosure can be suitably produced by a production method of obtaining an amide compound or a nitrogen-containing heterocyclic ring-containing compound by the production method described above, and then crosslinking the amide compound or the nitrogen-containing heterocyclic ring-containing compound. For the crosslinking, the amide compound, the nitrogen-containing heterocyclic ring-containing compound, or a mixture of the amide compound and the nitrogen-containing heterocyclic ring-containing compound can be used. The amide compound or the amide compound in the mixture is cyclodehydrated at the same time as the crosslinking to give a crosslinked product having the same structure as the crosslinked product obtained by crosslinking the nitrogen-containing heterocyclic ring-containing compound.

The amide compound of the present disclosure is a thermally crosslinkable amide compound, and the nitrogen-containing heterocyclic ring-containing compound of the present disclosure is a thermally crosslinkable nitrogen-containing heterocyclic ring compound. Therefore, the crosslinking can be performed by heating the amide compound or the nitrogen-containing heterocyclic ring-containing compound. The heating temperature for the crosslinking is preferably 250 to 550°C, and more preferably 320 to 400°C. The heating time is preferably 0.1 to 5 hours, and more preferably 0.5 to 4 hours. During the crosslinking, the amide compound or nitrogen-containing heterocyclic ring-containing compound may be heated so as to slowly raise the temperature of the amide compound or nitrogen-containing heterocyclic ring-containing compound. The temperature-increasing rate may be 1 to 10°C/min. For example, the amide compound or nitrogen-containing heterocyclic ring-containing compound may be heated to 300 to 330°C, and then heated at a rate of 1 to 10°C/min to raise the temperature by 30°C or more.

Prior to the crosslinking, the amide compound or nitrogen-containing heterocyclic ring-containing compound may be molded into a desired shape. The molding can be performed by a known method, such as compression molding. The amide compound or nitrogen-containing heterocyclic ring-containing compound may be molded by heating and melting, or may be molded by preparing a solution containing the amide compound or the nitrogen-containing heterocyclic ring-containing compound, and impregnating a substrate with the obtained solution or coating the obtained solution on the substrate.

Prior to the crosslinking, the amide compound or nitrogen-containing heterocyclic ring-containing compound may be mixed with a filler. By mixing with a filler, a crosslinked product containing a filler can be obtained. Further, prior to the crosslinking, the amide compound or nitrogen-containing heterocyclic ring-containing compound may be mixed with ordinary additives that are blended in rubber, for example, a processing aid, a plasticizer, a coloring agent, and the like.

In the case of mixing the amide compound or nitrogen-containing heterocyclic ring-containing compound with a filler or an additive, a conventional polymer processing machine, for example, an open roll, a Banbury mixer, a kneader, a closed mixer, and the like can be used.

Since the crosslinked product of the present disclosure exhibits elastomeric characteristics at a high temperature, and moreover in a wide temperature range, the crosslinked product of the present disclosure can be suitably used as a member of a semiconductor manufacturing apparatus that in particular requires heat resistance, in particular as a sealing material of a semiconductor manufacturing apparatus. Examples of the sealing material include O-rings, square-rings, gaskets, packings, oil seals, bearing seals, lip seals, and the like.

In addition, the crosslinked product of the present disclosure can also be used as various polymer products used in a semiconductor manufacturing apparatus, such as diaphragms, tubes, hoses, various rubber rolls, and belts. Further, the crosslinked product of the present disclosure can also be used as a coating material and a lining material.

As used herein, the semiconductor manufacturing apparatus referred to in the present disclosure is not limited to an apparatus for manufacturing semiconductors, and widely includes general manufacturing apparatus used in the semiconductor field requiring a high level of cleanliness, such as apparatus for manufacturing liquid crystal panels and plasma panels. Examples thereof include the following.

(1) Etching apparatus

Dry etching apparatus

Plasma etching apparatus
Reactive ion etching apparatus
Reactive ion beam etching apparatus
Sputter etching apparatus
Ion beam etching apparatus
Wet etching apparatus
Ashing apparatus

(2) Cleaning apparatus dry etching cleaning apparatus

$UV/O_3$ cleaning apparatus
Ion beam cleaning apparatus
Laser beam cleaning apparatus
Plasma cleaning apparatus
Gas etching cleaning apparatus
Extraction cleaning apparatus
Soxhlet extraction cleaning apparatus
High-temperature high-pressure extraction cleaning apparatus
Microwave extraction cleaning apparatus
Supercritical extraction cleaning apparatus

(3) Exposure apparatus

Stepper
Coater/developer

(4) Polishing apparatus
CMP apparatus
(5) Film formation apparatus

CVD apparatus
Sputtering apparatus

(6) Diffusion/ion implantation apparatus

Oxidation diffusion apparatus
Ion implantation apparatus

The crosslinked product of the present disclosure exhibits excellent performance as a sealing material for, for example, a CVD apparatus, a plasma etching apparatus, a reactive ion etching apparatus, an ashing apparatus, or an excimer laser exposure apparatus.

Further, a solution in which the amide compound or nitrogen-containing heterocyclic ring-containing compound of the present disclosure is dissolved in a solvent can be used as a coating material. Since the amide compound or nitrogen-containing heterocyclic ring-containing compound of the present disclosure has excellent heat resistance and adhesiveness to a substrate, the amide compound or nitrogen-containing heterocyclic ring-containing compound can be suitably applied for coating the surface of the substrate. For example, a heat-resistant coating can be formed by applying a solution in which the amide compound or nitrogen-containing heterocyclic ring-containing compound of the present disclosure is dissolved in a solvent to the surface of a substrate and drying by heating.

Examples of the substrate include a metal substrate, a glass substrate, a resin substrate, a rubber substrate, and the like. From the perspective of obtaining a particularly high adhesiveness, a metal substrate is preferable. Examples of the metal substrate include a substrate made of iron, copper, stainless steel, aluminum, brass, and the like.

Further, a solution containing a filler in addition to the amide compound or nitrogen-containing heterocyclic ring-containing compound of the present disclosure may be prepared and applied to the substrate. Examples of the filler include those described above. By using a filler, various physical properties such as heat resistance, hardness, and abrasion resistance of the heat-resistant coating can be improved.

A solution containing another polymer in addition to the amide compound or nitrogen-containing heterocyclic ring-containing compound of the present disclosure may be prepared and applied to the substrate. As the other polymer, a polymer having excellent heat resistance is preferable. Examples thereof include polyether ether ketone, polyphenylene

sulfide, polyphenylsulfone, polysulfone, polyamideimide, polybenzoxazole, polybenzimidazole, polyimide, and the like. The content of the amide compound or nitrogen-containing heterocyclic ring-containing compound of the present disclosure may be, for example, 0.0001 to 10,000 parts by mass based on 100 parts by mass of the other polymer.

Further, a fiber-reinforced composite material can be obtained by impregnating a fibrous substrate with a solution in which the amide compound or nitrogen-containing heterocyclic ring-containing compound of the present disclosure is dissolved in a solvent.

Further, a solution in which the amide compound or nitrogen-containing heterocyclic ring-containing compound of the present disclosure is dissolved in a solvent can be used as an adhesive. By using an adhesive containing the amide compound or nitrogen-containing heterocyclic ring-containing compound of the present disclosure, an adhesive layer having excellent heat resistance can be formed.

The solvent for dissolving the amide compound or nitrogen-containing heterocyclic ring-containing compound of the present disclosure is not limited, and examples thereof include dimethyl sulfoxide (DMSO), N,N-dimethylformamide (DMF), N,N-dimethylacetamide (DMAc), N-methyl-2-pyrrolidone (NMP), 1,3-dimethylimidazolidone (DMI), sulfolane, tetrahydrofuran (THF), and acetone. Of these, N-methyl-2-pyrrolidone (NMP) and 1,3-dimethylimidazolidone (DMI) are preferable.

Embodiments of the present disclosure have been described above, but it will be understood that various modifications can be made to the embodiments and details thereof without departing from the gist and scope of the claims.

## EXAMPLES

Next, the embodiments of the present disclosure will be described with reference to examples, but the present disclosure is not limited to such examples.

The numerical values in the examples were measured by the following methods.

(1) GPC: High-speed GPC System HLC-8220GPC, manufactured by Tosoh Corporation (column: Tosoh TSKgel ($\alpha$-M), column temperature: 45°C, detector: UV-8020, wavelength 254 nm, eluent: N-methyl-2-pyrrolidone (NMP) (including 0.01 mol/L lithium bromide), calibration curve: standard polystyrene, column flow velocity: 0.2 mL/min)
(2) Infrared spectrum (FT-IR): FT/IR-4200, manufactured by JASCO Corporation
(3) Nuclear magnetic resonance spectrum (NMR) : AC400P, manufactured by BRUKER
(4) Thermogravimetric analysis (TGA): TG/DTA7300, manufactured by Hitachi High-Tech Science Corporation, temperature-increasing rate 10°C/min
(5) Differential scanning calorimetry (DSC): DSC7000, manufactured by Hitachi High-Tech Science Corporation, temperature-increasing rate 10°C/min
(6) Thermomechanical analysis (TMA) : IMA7000, manufactured by Hitachi High-Tech Science Corporation, temperature-increasing rate 10°C/min
(7) Dynamic viscoelasticity measurement (DMA): DMA7100, manufactured by Hitachi High-Tech Science Corporation, temperature-increasing rate 2°C/min
(8) Tensile test: Autograph AGS-D type, manufactured by Shimadzu Corporation, tensile speed 10 mm/min
(9) Dielectric constant measurement: Dielectric constant / dielectric tangent measuring device (cavity resonator type, 20 GHz) manufactured by AET

<Synthesis example 1>

1,6-Bis(4-aminophenyl)dodecafluorohexane (APDF6)

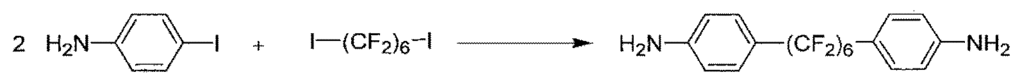

4-iodoaniline (15 g, 69 mmol) and DMSO (60 mL) were charged into a 100 mL eggplant flask equipped with a stirrer, a Dimroth condenser, and a three-way cock, and made to dissolve. Then, the mixture was quickly charged with copper powder (21.6 g, 342 mmol) so as not to oxidize in air, and quickly charged with 1,6-diiodoperfluorohexane (18.9 g, 33 mmol) before it deliquesced. Then, the temperature was raised to 120°C, and the mixture was reacted while stirring for 24 hours at 120°C. After completion of the reaction, the mixture was cooled to room temperature and suction filtered to recover the DMSO solution. The DMSO was distilled off from the DMSO solution by vacuum distillation to obtain a crude product. The crude product was dissolved in dichloromethane, washed with distilled water, the dichloromethane

solution was recovered, and the resulting product was dried over anhydrous sodium sulfate overnight. The dichloromethane was distilled off with an evaporator to obtain a yellow crude product. The yellow crude product was recrystallized from hexane, and dried under reduced pressure at room temperature. The product was dissolved in methanol and decolorized with activated carbon. After removing the methanol, the product was dried under reduced pressure at 60°C overnight to obtain APDF6 (yield amount: 10.8 g, yield: 65%) in the form of a yellow powder.

The structure was confirmed by FT-IR, $^1$H-NMR, $^{13}$C-NMR, $^{19}$F-NMR, and elemental analysis.

FT-IR (KBr, $cm^{-1}$): 3444 (N-H), 3057 (C-H), 1609 (C=C), 1185 (C-F)
$^1$H-NMR (DMSO-$d_6$, ppm) : 5.82 (s, 4H, $NH_2$), 6.62 (d, 4H, Ar-H), 7.20 (d, 4H, Ar-H)
$^{13}$C-NMR (DMSO-$d_6$, ppm): 113.0, 127.6, 152.4
$^{19}$F-NMR (DMSO-$d_6$, ppm) : -122.2, -121.5, -108.0
Melting point: 79.4°C (DSC)
Elemental analysis: Calculated C, 44.64%; H, 2.50%; N, 5.78%
Measured C, 44.66%; H, 2.72%; N, 5.49%

<Synthesis example 2>

1,8-Bis(4-aminophenyl)hexadecafluorooctane (APHF8)

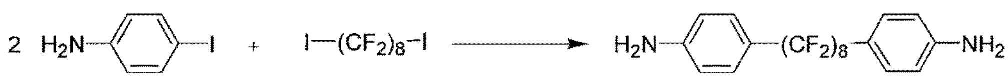

4-iodoaniline (7.5 g, 34 mmol) and DMSO (20 mL) were charged into a 100 mL eggplant flask equipped with a stirrer, a Dimroth condenser, and a three-way cock, and made to dissolve. Then, the mixture was quickly charged with copper powder (10 g, 172 mmol) so as not to oxidize in air, and quickly charged with 1,8-diiodoperfluorooctane (11.6 g, 17 mmol) before it deliquesced. Then, the temperature was raised to 120°C, and the mixture was reacted while stirring for 24 hours at 120°C. After completion of the reaction, the mixture was cooled to room temperature and suction filtered to recover the DMSO solution. The DMSO was distilled off from the DMSO solution by vacuum distillation to obtain a crude product. The crude product was dissolved in dichloromethane, washed with distilled water, the dichloromethane solution was recovered, and the resulting product was dried over anhydrous sodium sulfate overnight. The dichloromethane was distilled off with an evaporator, and 8.4 g (80%) of a crude product was collected. The crude product was recrystallized from hexane, and dried under reduced pressure at room temperature. The product was dissolved in methanol and decolorized with activated carbon. The methanol was removed to obtain APHF8 (yield amount: 5.9 g, yield: 58%) in the form of a yellow powder.

The structure was confirmed by FT-IR, $^1$H-NMR, $^{13}$C-NMR, and $^{19}$F-NMR. FT-IR (KBr, $cm^{-1}$): 3489-3392 (N-H), 3050 (C-H), 1581 (C=C), 1259-1145 (C-F) $^1$H-NMR (DMSO-$d_6$, ppm): 5.86 (s, 4H, $NH_2$), 6.66 (d, 4H, Ar-H), 7.22 (d, 4H, Ar-H)

$^{13}$C-NMR (DMSO-$d_6$, ppm): 113.0, 127.8, 152.4
$^{19}$F-NMR (DMSO-$d_6$, ppm): -121.7, -121.1, -107.5
Melting point: 85.8°C (DSC)
Elemental analysis: Calculated C, 41.16%; H, 2.08%; N, 4.80%
Measured C, 41.23%; H, 2.28%; N, 4.85%

<Synthesis example 3>

1,6-Bis(3-aminophenyl)dodecafluorohexane (mAPDF6)

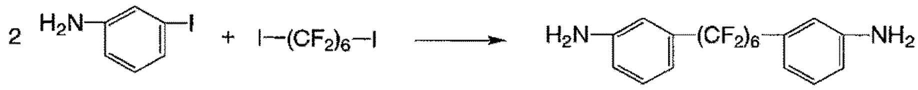

3-iodoaniline (5.0 g, 22.8 mmol) and DMSO (20 mL) were charged into a 100 mL eggplant flask equipped with

a stirrer, a Dimroth condenser, and a three-way cock, and made to dissolve. Then, the mixture was quickly charged with copper powder (7.2 g, 114 mmol) so as not to oxidize in air, and quickly charged with 1,6-diiodoperfluorohexane (6.3 g, 11.4 mmol) before it deliquesced. Then, the temperature was raised to 120°C, and the mixture was reacted while stirring for 24 hours at 120°C. After completion of the reaction, the mixture was cooled to room temperature and suction filtered to recover the DMSO solution. The DMSO was distilled off from the DMSO solution by vacuum distillation to obtain a crude product. The crude product was dissolved in dichloromethane, washed with distilled water, the dichloromethane solution was recovered, and the resulting product was dried over anhydrous sodium sulfate overnight. The dichloromethane was distilled off with an evaporator to obtain a yellow crude product. The yellow crude product was recrystallized from a mixed solvent of methanol / distilled water, and dried under reduced pressure at 60°C. The product was dissolved in methanol and decolorized with activated carbon. After removing the methanol, the product was dried under reduced pressure at 60°C overnight to obtain mAPDF6 (yield amount: 3.0 g, yield: 54%) in the form of a yellow powder.

The structure was confirmed by $^1$H-NMR, $^{13}$C-NMR, $^{19}$F-NMR, and elemental analysis.

$^1$H-NMR ($CDCl_3$, ppm) : 3.81 (s, 4H, $NH_2$), 6.83 (d, 2H, Ar-H), 6.85 (s, 2H, Ar-H), 6.95 (d, 2H, Ar-H), 7.24 (t, 2H, Ar-H)
$^{13}$C-NMR ($CDCl_3$, ppm): 113.1, 116.9, 118.2, 129.6, 130.3, 146.7
$^{19}$F-NMR ($CDCl_3$, ppm): -121.9, -121.3, -110.7
Melting point: 79.4°C (DSC)
Elemental analysis: Calculated C, 44.64%; H, 2.50%; N, 5.78%
Measured C, 44.75%; H, 2.80%; N, 5.52%

<Synthesis example 4>

1,6-Bis(p-tolyl)dodecafluorohexane (TDF6)

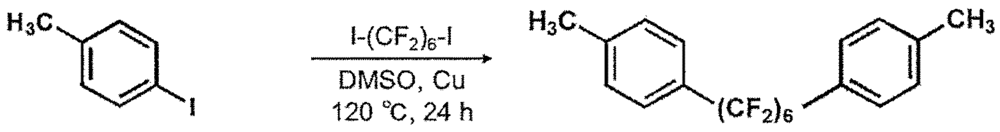


4-Iodotoluene (8.72 g, 40 mmol) and DMSO (20 mL) were charged into a 100 mL three-necked flask equipped with a stirrer, a condenser, and a nitrogen introduction tube, and made to dissolve. Next, 1,6-diiodoperfluorohexane (11.08 g, 20 mmol) and copper powder (12.69 g, 200 mmol) were added, the temperature was increased in steps to 120°C, and the mixture was reacted for 24 hours at 120°C. After completion of the reaction, the mixture was allowed to cool to room temperature, the copper powder was removed by suction filtration, and the DMSO was distilled off by vacuum distillation. The obtained crude product was dissolved in diethyl ether (100 mL), and washed with distilled water. The organic layer was separated and dehydrated overnight with anhydrous sodium sulfate. The diethyl ether was distilled off with an evaporator to obtain a white product (TDF6) (yield amount: 8.59 g, yield: 89%).

The physical properties of the obtained product are shown below.

$^1$H-NMR ($CDCl_3$, ppm): 2.34 (s, 6H, $CH_3$), 7.41 (d, 4H, Ar-H), 7.51 (d, 4H, Ar-H)
$^{13}$C-NMR ($CDCl_3$, ppm): 20.86, 126.46, 129.67, 136.58, 136.67, 138.60, 138.71, 142.90
$^{19}$F-NMR ($CDCl_3$, ppm): -109.72, -121.57, -122.13

<Synthesis example 5>

1,6-Bis(4-carboxyphenyl)dodecafluorohexane (CPDF6)

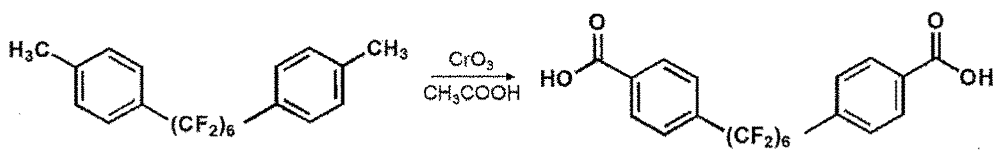

TDF6 (14.47 g, 30 mmol), acetic acid (150 mL), and concentrated sulfuric acid (20 mL) were charged into a 500 mL three-necked flask equipped with a stirrer, a nitrogen introduction tube, a thermometer, and a dropping funnel, made to dissolve, and the resulting mixture was cooled to 0°C. A solution prepared by dissolving chromium oxide (10.00 g, 100 mmol) in acetic anhydride (50 mL) was added dropwise, and after the addition was completed, the mixture was reacted for 12 hours at room temperature while stirring. After completion of the reaction, the reaction solution was charged into distilled water (1 L) to precipitate a product. The product was collected by suction filtration, and dried under reduced pressure for 12 hours at 100°C. The yield amount obtained was 15.9 g (98% yield). The dried product was recrystallized from a mixed solvent of DMF / distilled water, and dried under reduced pressure for 12 hours at 100°C to obtain a product in the form of a white powder (yield amount: 6.35 g, yield: 39%) .

The physical properties of the obtained product are shown below.

$^1$H-NMR (DMSO-$d_6$, ppm) : 7.80 (d, 4H, Ar-H), 8.13 (d, 4H, Ar-H), 13.50 (s, 1H, COOH)
$^{13}$C-NMR (DMSO-$d_6$, ppm) : 127.15, 129.93, 131.19, 134.74, 136.57, 136.66, 138.70, 166.23
$^{19}$F-NMR (DMSO-$d_6$, ppm) : -110.41, -121.57, -122.04

<Synthesis example 6>

1,6-Bis(4-chlorocarbonylphenyl)dodecafluorohexane (CCPDF6)

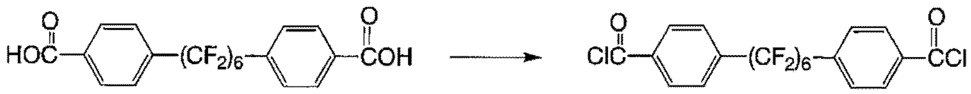

CPDF6 (5.38 g, 9.9 mmol) and thionyl chloride (40 mL) were charged into an eggplant flask (100 mL) equipped with a stirrer, a condenser, and a calcium chloride tube, the temperature was slowly raised to 85°C, and the mixture was stirred for 1 hour. The mixture was allowed to cool to room temperature, and excess thionyl chloride was distilled off by vacuum distillation to obtain a solid product. The obtained solid product was purified by sublimation (140°C/0.8 Torr) to obtain a white needle-like crystal product (yield amount: 4.4 g, yield: 77%).

The structure was confirmed by FT-IR, $^1$H-NMR, $^{13}$C-NMR, $^{19}$F-NMR, and elemental analysis.

FT-IR (KBr, $cm^{-1}$): 3063 (Ar-H), 1746 (C=O), 1142 (C-F)
$^1$H-NMR ($CDCl_3$, ppm): 8.24 (d, 4H, Ar-H), 7.75 (d, 4H, Ar-H)
$^{13}$C-NMR ($CDCl_3$, ppm): 127.78, 131.40, 135.49, 136.44, 167.76,
$^{19}$F-NMR ($CDCl_3$, ppm): -111.33, -121.10, -121.60
Melting point: 129 to 130°C
Elemental analysis: Calculated C, 41.48%; H, 1.39%
Measured C, 41.43%; H, 1.48%

<Synthesis example 7>

Bis(4,6-dimethoxy-1,3,5-triazin-2-yl)-p-dodecafluorohexylenedibenzoate (p-DFBBT)

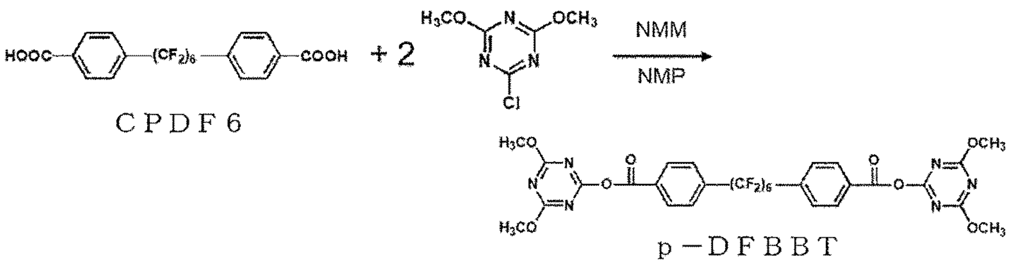


CPDF6 (27.11 g, 50 mmol), chlorodimethoxytriazine (CDMT) (17.51 g, 100 mmol), and NMP (400 mL) were charged into a 500 mL three-necked flask equipped with a stirrer, a nitrogen introduction tube, and a thermometer, made

to dissolve, and the resulting mixture was cooled to 0°C. Then, N-methylmorpholine (NMM) (11.59 mL, 110 mmol) was added, and the mixture was reacted for 1 hour at 0°C. After completion of the reaction, the reaction solution was charged into 400 mL of an aqueous solution adjusted to pH 3 using acetic acid to precipitate a product. The product was collected by suction filtration and dried under reduced pressure for 12 hours at 50°C. The crude yield amount was 32.8 g (crude yield 80%). The dried crude product was recrystallized from a mixed solvent of chloroform / hexane, and dried under reduced pressure for 12 hours at 50°C to obtain a product in the form of a white powder (yield amount: 21.33 g, yield: 52%).

The physical properties of the obtained product are shown below.

$^1$H-NMR ($CDCl_3$, ppm): 4.09 (s, 12H, $CH_3$), 7.76 (d, 4H, Ar-H), 8.30 (s, 4H, Ar-H)
$^{13}$C-NMR ($CDCl_3$, ppm): 56.20, 127.64, 130.90, 131.61, 137.01, 137.10, 139.19, 161.54, 170.70, 171.13, 174.39
$^{19}$F-NMR ($CDCl_3$, ppm): -112.65, -122.53, -123.10

<Experiment example 1>

Amide compound (6FTA-*p*-DFBBT)

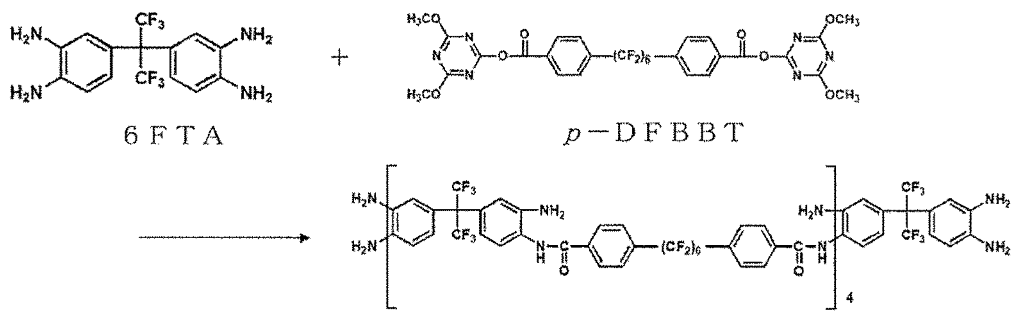


2,2-Bis(3,4-diaminophenyl)hexafluoropropane (6FTA) (1.82 g, 5 mmol) and NMP (9 mL) were charged into a 100 mL three-necked flask equipped with a stirrer and a nitrogen introduction tube, made to dissolve, and the resulting mixture was cooled to 0°C. Next, a triazine-based active diester (p-DFBBT) (3.28 g, 4 mmol) was added so that the number of repeating units (n) of the amide compound was 4, and the mixture was reacted for 1 hour at 0°C. Then, the mixture was reacted for 12 hours at room temperature. After completion of the reaction, the polymerization solution was charged into distilled water (300 mL) to precipitate an amide compound. The precipitated amide compound was collected by suction filtration, and dried under reduced pressure at room temperature for 12 hours. The crude yield was 98%. The dried polymer was dissolved in NMP (9 mL) and purified by reprecipitation with distilled water to obtain an amide compound (6FTA-p-DFBBT) in the form of a pale yellow powder (yield amount: 3.11 g, yield: 81%).

The physical properties of the obtained amide compound are shown below.

FT-IR (KBr, $cm^{-1}$): 1,658 (C=O), 1,500 (C=C), 1,170 (C-F)
Number average molecular weight ($M_n$): 3,600 (calculated value 3,700), molecular weight distribution ($M_w$ / $M_n$): 2.2
Solubility: Dissolved in NMP, DMAc, DMF, DMSO, THF, acetone, methanol at room temperature

<Experiment example 2>

Amide compound (6FTAPh-p-DFBBT)

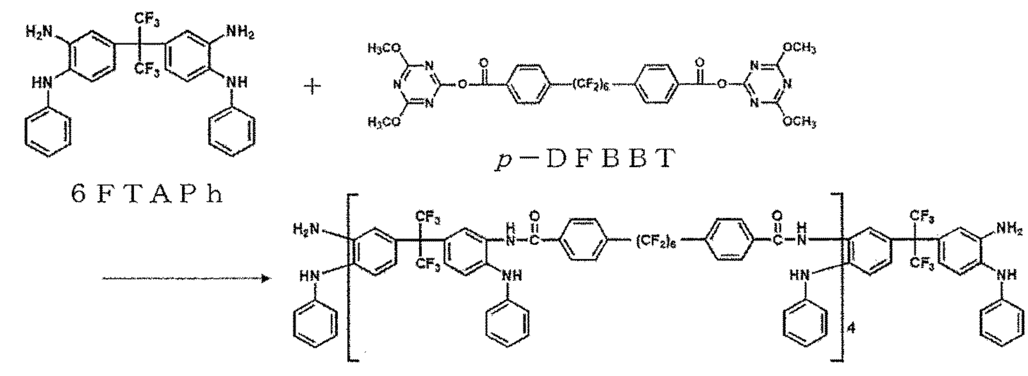

6FTAPh

p-DFBBT

6FTAPh (2.58 g, 5 mmol) and NMP (9 mL) were charged into a 100 mL three-necked flask equipped with a stirrer and a nitrogen introduction tube, and made to dissolve. Next, a triazine-based active diester (p-DFBBT) (3.28 g, 4 mmol) was added so that the number of repeating units (n) of the amide compound was 4, and the mixture was reacted for 12 hours at room temperature. After completion of the reaction, the polymerization solution was charged into distilled water (300 mL) to precipitate an amide compound. The precipitated amide compound was collected by suction filtration, and dried under reduced pressure at room temperature for 12 hours. The crude yield was 94%. The dried amide compound was dissolved in NMP (9 mL) and purified by reprecipitation with distilled water to obtain an amide compound in the form of a white powder (6FTAPh-*p*-DFBBT) (yield amount: 3.44 g, yield: 77%).

The physical properties of the obtained amide compound are shown below.

FT-IR (KBr, $cm^{-1}$): 1,661 (C=O), 1,598 (C=C), 1,169 (C-F)
Logarithmic viscosity: 0.25 dL/g (NMP solution having a 0.5 g/dL concentration, measured at 30°C)
Number average molecular weight ($M_n$): 4,500 (calculated value 4,600), molecular weight distribution ($M_w$ / $M_n$): 2.0
Solubility: Dissolved in NMP, DMAc, DMF, DMSO, THF, chloroform, acetone, methanol at room temperature

<Example 1>

Amide compound (6FTA-p-DFBBT-PEPA)

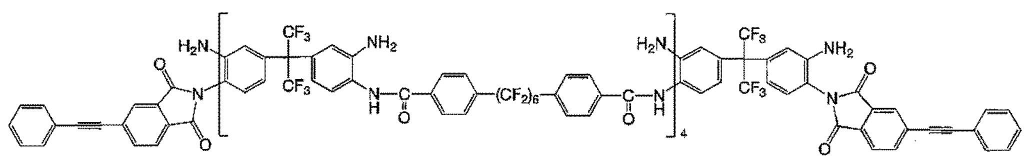

An amide compound (6FTA-*p*-DFBBT) (0.371 g, 0.1 mmol) and NMP (1.6 mL) were charged into a 100 mL three-necked flask equipped with a stirrer and a nitrogen introduction tube, and made to dissolve. Next, 4-phenylethynylphthalic anhydride (PEPA) (0.050 g, 0.2 mmol) as a cross-linking agent was added, and the mixture was reacted for 12 hours at room temperature. After completion of the reaction, the polymerization solution was coated on a glass plate, dried under reduced pressure for 6 hours at room temperature, 6 hours at 60°C, 6 hours at 100°C, 1 hour at 150°C, 1 hour at 200°C, and 1 hour at 250°C, to obtain an amide compound having a crosslinkable group introduced at an end.

The physical properties of the obtained amide compound are shown below.

FT-IR (KBr, $cm^{-1}$): 2,214 (C≡C), 1,725 (C=O), 1,657 (C=O), 1,617 (C=C), 1,185 (C-F)
Solubility: Dissolved in NMP, DMAc, and DMF at room temperature
Glass transition temperature: 250°C (DSC measurement)
5% Weight loss temperature: 429°C (in air), 433°C (in nitrogen) (TGA)
10% Weight loss temperature: 496°C (in air), 504°C (in nitrogen) (TGA)
Carbonization yield: 57% (800°C in nitrogen) (TGA)

<Example 2>

Nitrogen-containing heterocyclic ring-containing compound (imide compound: APDF6-6FDA-PEPA)

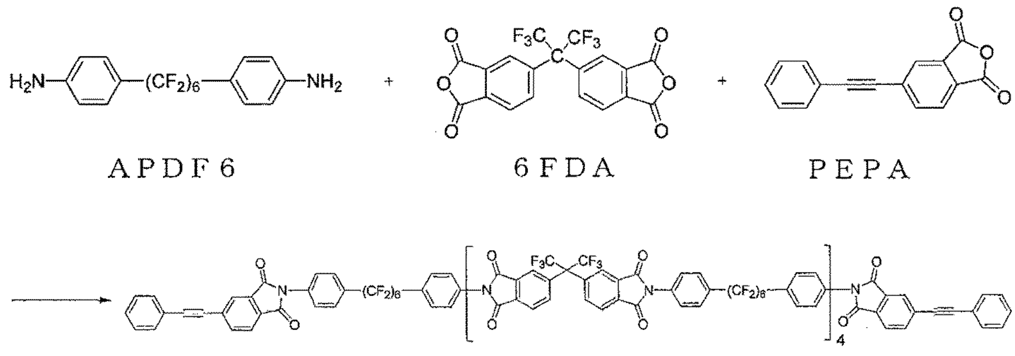

APDF6 6FDA PEPA

A 100 mL three-necked flask equipped with a stirrer, a Dimroth condenser, a flat stopper, and a nitrogen introduction tube was heated and dried with a burner. Then, APDF6 (1.211 g, 2.50 mmol) and distilled NMP (7.0 mL) were charged into the flask, and the mixture was completely dissolved by stirring. Then, 4,4'- (hexafluoroisopylidene) diphthalic acid dianhydride (6FDA) (0.888 g, 2.00 mmol) was added, and the mixture was placed in an ice bath and stirred for 1 hour at 0 to 5°C. Then, after gradually raising the temperature and stirring for 6 hours at 60°C, 4-phenylethynylphthalic anhydride (PEPA) (0.248 g, 1.00 mmol) as a cross-linking agent was added, and the mixture was stirred for 15 hours at 60°C. After cooling to room temperature, the polymerization solution was cast on a glass plate, and dried under reduced pressure for 3 hours at room temperature to obtain a precursor amide compound. Then, the precursor amide compound was dried under reduced pressure in a program consisting of 1 hour at 80°C, 1 hour at 100°C, 1 hour at 150°C, 1 hour at 200°C, and 1 hour at 250°C to obtain a nitrogen-containing heterocyclic ring-containing compound (imide compound) in the form of a yellow powder.

The physical properties of the obtained nitrogen-containing heterocyclic ring-containing compound (imide compound) are shown below.

FT-IR (KBr, $cm^{-1}$): 2,214 (C≡C), 1,787 (C=O), 1,727 (C=O), 1,373 (C-N), 1,298 (C-F), 745 (imide ring)
Logarithmic viscosity ($\eta_{inh}$): 0.14 dL/g (NMP solution having a 0.5 g/dL concentration, measured at 30°C)
Number average molecular weight (Mn): 4,700 (calculated value 4,500), molecular weight distribution (Mw/Mn): 2.4
Solubility: Dissolved in NMP, EMSO, DMAc, DMF, DMI, γ-butyrolactone, THF, and chloroform at room temperature
5% Weight loss temperature: 522°C (in air), 526°C (in nitrogen) (TGA)
10% Weight loss temperature: 547°C (in air), 551°C (in nitrogen) (TGA)
Carbonization yield: 50% (800°C in nitrogen) (TGA)
Glass transition temperature: 171°C (DSC)

<Example 3>

Nitrogen-containing heterocyclic ring-containing compound (imide compound: APDF6-ODPA-PEPA)

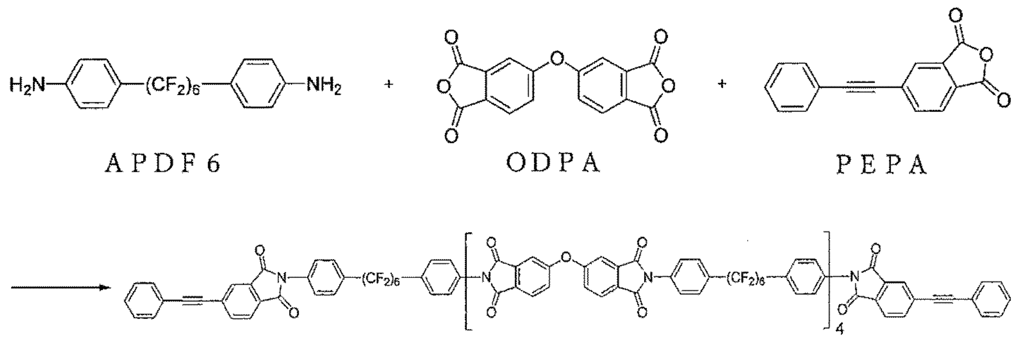

APDF6 ODPA PEPA

A 100 mL three-necked flask equipped with a stirrer, a Dimroth condenser, a flat stopper, and a nitrogen introduction tube was heated and dried with a burner. Then, APDF6 (1.211 g, 2.50 mmol) and distilled NMP (7.7 mL) were charged into the flask, and the mixture was completely dissolved by stirring. Then, oxydiphthalic acid dianhydride (ODPA) (0.620 g, 2.00 mmol) was added, and the mixture was placed in an ice bath and stirred for 1 hour at 0 to 5°C. Then, after gradually raising the temperature and stirring for 6 hours at 60°C, 4-phenylethynylphthalic anhydride (PEPA) (0.248 g, 1.00 mmol) as a cross-linking agent was added, and the mixture was stirred for 12 hours at 60°C. After cooling to room temperature, the polymerization solution was cast on a glass plate, and dried under reduced pressure for 3 hours at room temperature to obtain a precursor amide compound. Then, the precursor amide compound was dried under reduced pressure in a program consisting of 1 hour at 80°C, 1 hour at 100°C, 1 hour at 150°C, 1 hour at 200°C, and 1 hour at 250°C to obtain a nitrogen-containing heterocyclic ring-containing compound (imide compound) in the form of a yellow powder.

The physical properties of the obtained nitrogen-containing heterocyclic ring-containing compound (imide compound) are shown below.

FT-IR (KBr, $cm^{-1}$): 2,213 (C≡C), 1,785 (C=O), 1,727 (C=O), 1,370 (C-N), 1,275 (C-F), 743 (imide ring)
Logarithmic viscosity ($\eta_{inh}$): 0.19 dL/g (NMP solution having a 0.5 g/dL concentration, measured at 30°C)
Number average molecular weight (Mn): 4,100 (calculated value 4,000), molecular weight distribution (Mw/Mn): 4.0
Solubility: Dissolved in NMP, DMAc, and DMI at room temperature
5% Weight loss temperature: 504°C (in air), 523°C (in nitrogen) (TGA)
10% Weight loss temperature: 539°C (in air), 550°C (in nitrogen) (TGA)
Carbonization yield: 51% (800°C in nitrogen) (TGA)
Glass transition temperature: 118°C (DSC)

<Example 4>

Nitrogen-containing heterocyclic ring-containing compound (imide compound: APDF6-BPDA-PEPA)

A nitrogen-containing heterocyclic ring-containing compound (imide compound) in the form of a yellow powder was obtained in the same manner using 3,3',4,4'-biphenyltetracarboxylic acid dianhydride (BPDA) instead of the ODPA of Example 3.

The physical properties of the obtained nitrogen-containing heterocyclic ring-containing compound (imide compound) are shown below.

FT-IR (KBr, $cm^{-1}$): 2,214 (C≡C), 1,787 (C=O), 1,727 (C=O), 1,373 (C-N), 1,298 (C-F), 745 (imide ring)
Logarithmic viscosity ($\eta_{inh}$): 0.21 dL/g (NMP solution having a 0.5 g/dL concentration, measured at 30°C)
5% Weight loss temperature: 518°C (in air), 534°C (in nitrogen) (TGA)
10% Weight loss temperature: 557°C (in air), 562°C (in nitrogen) (TGA)
Carbonization yield: 53% (800°C in nitrogen) (TGA)
Glass transition temperature: 128°C (DSC)

<Example 5>

Nitrogen-containing heterocyclic ring-containing compound (imide compound: APHF8-6FDA-PEPA)

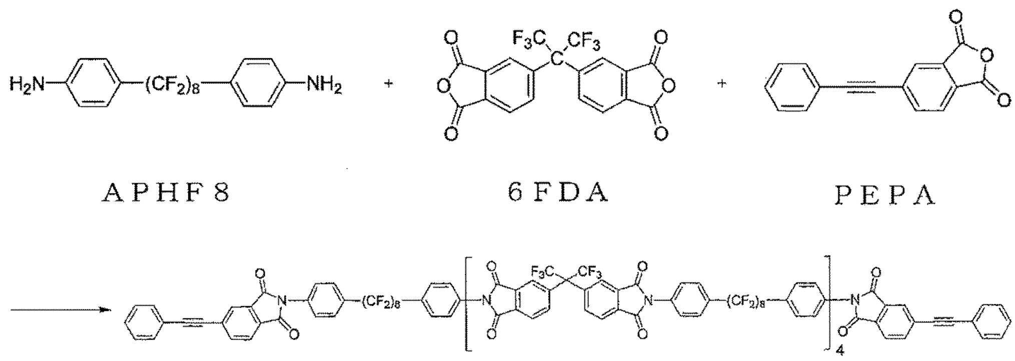

APHF8 6FDA PEPA

A 100 mL three-necked flask equipped with a stirrer, a Dimroth condenser, a flat stopper, and a nitrogen introduction tube was heated and dried with a burner. Then, APHF8 (1.461 g, 2.50 mmol) and distilled NMP (9.7 mL) were charged into the flask, and the mixture was completely dissolved by stirring. Then, 4,4'- (hexafluoroisopylidene) diphthalic acid dianhydride (6FDA) (0.888 g, 2.00 mmol) was added, and the mixture was placed in an ice bath and stirred for 1 hour at 0 to 5°C. Then, after gradually raising the temperature and stirring for 6 hours at 60°C, 4-phenylethynylphthalic anhydride (PEPA) (0.248 g, 1.00 mmol) as a cross-linking agent was added, and the mixture was stirred for 15 hours at 60°C. After cooling to room temperature, the polymerization solution was cast on a glass plate, and dried under reduced pressure for 3 hours at room temperature to obtain a precursor amide compound. Then, the precursor amide compound was dried under reduced pressure in a program consisting of 1 hour at 80°C, 1 hour at 100°C, 1 hour at 150°C, 1 hour at 200°C, and 1 hour at 250°C to obtain a nitrogen-containing heterocyclic ring-containing compound (imide compound) in the form of a yellow powder.

The physical properties of the obtained nitrogen-containing heterocyclic ring-containing compound (imide compound) are shown below.

FT-IR (KBr, $cm^{-1}$): 2,208 (C≡C), 1,785 (C=O), 1,728 (C=O), 1,370 (C-N), 1,258 (C-F), 745 (imide ring)
Logarithmic viscosity ($\eta_{inh}$): 0.14 dL/g (NMP solution having a 0.5 g/dL concentration, measured at 30°C)
Number average molecular weight (Mn): 4,900 (calculated value 4,500), molecular weight distribution (Mw/Mn): 2.3
Solubility: Dissolved in NMP, DMAc, DMF, DMI, γ-butyrolactone, THF, and chloroform at room temperature
5% Weight loss temperature: 526°C (in air), 537°C (in nitrogen) (TGA)
10% Weight loss temperature: 557°C (in air), 558°C (in nitrogen) (TGA)
Carbonization yield: 49% (800°C in nitrogen) (TGA)
Glass transition temperature: 106°C (DSC)

<Example 6>

Nitrogen-containing heterocyclic ring-containing compound (imide compound: APHF8-ODPA-PEPA)

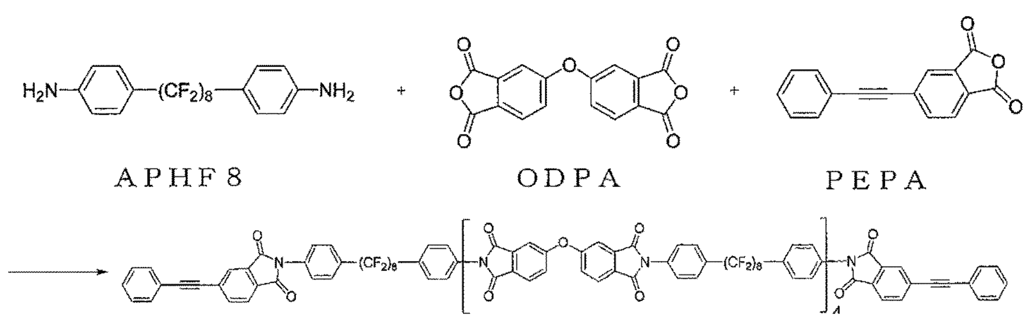

APHF8 ODPA PEPA

A 100 mL three-necked flask equipped with a stirrer, a Dimroth condenser, a flat stopper, and a nitrogen introduction tube was heated and dried with a burner. Then, APHF8 (1.461 g, 2.50 mmol) and distilled NMP (8.7 mL) were charged into the flask, and the mixture was completely dissolved by stirring. Then, oxydiphthalic acid dianhydride (ODPA) (0.620 g, 2.00 mmol) was added, and the mixture was placed in an ice bath and stirred for 1 hour at 0 to 5°C. Then, after gradually raising the temperature and stirring for 6 hours at 60°C, 4-phenylethynylphthalic anhydride (PEPA)

(0.248 g, 1.00 mmol) as a cross-linking agent was added, and the mixture was stirred for 15 hours at 60°C. After cooling to room temperature, the polymerization solution was cast on a glass plate, and dried under reduced pressure for 3 hours at room temperature to obtain a precursor amide compound. Then, the precursor amide compound was dried under reduced pressure in a program consisting of 1 hour at 80°C, 1 hour at 100°C, 1 hour at 150°C, 1 hour at 200°C, and 1 hour at 250°C to obtain a nitrogen-containing heterocyclic ring-containing compound (imide compound) in the form of a yellow powder.

The physical properties of the obtained nitrogen-containing heterocyclic ring-containing compound (imide compound) are shown below.

FT-IR (KBr, $cm^{-1}$): 2,212 (C≡C), 1,785 (C=O), 1,727 (C=O), 1,375 (C-N), 1,276 (C-F), 744 (imide ring)
Logarithmic viscosity ($\eta_{inh}$): 0.15 dL/g (NMP solution having a 0.5 g/dL concentration, measured at 30°C)
5% Weight loss temperature: 520°C (in air), 529°C (in nitrogen) (TGA)
10% Weight loss temperature: 545°C (in air), 553°C (in nitrogen) (TGA)
Carbonization yield: 53% (800°C in nitrogen) (TGA)
Glass transition temperature: 156°C (DSC)

<Example 7>

Nitrogen-containing heterocyclic ring-containing compound (imide compound: mAPDF6- 6FDA- PEPA)

A nitrogen-containing heterocyclic ring-containing compound (imide compound) in the form of a yellow powder was obtained in the same manner using mAPDF6 instead of the APDF6 of Example 2.

The physical properties of the obtained nitrogen-containing heterocyclic ring-containing compound (imide compound) are shown below.

FT-IR (KBr, $cm^{-1}$): 2,214 (C≡C), 1,787 (C=O), 1,727 (C=O), 1,373 (C-N), 1,298 (C-F), 745 (imide ring)
Logarithmic viscosity ($\eta_{inh}$): 0.15 dL/g (NMP solution having a 0.5 g/dL concentration, measured at 30°C)
Number average molecular weight (Mn): 5,000 (calculated value 4,500), molecular weight distribution (Mw/Mn): 1.9
5% Weight loss temperature: 495°C (in air), 526°C (in nitrogen) (TGA)
10% Weight loss temperature: 527°C (in air), 552°C (in nitrogen) (TGA)
Carbonization yield: 54% (800°C in nitrogen) (TGA)
Glass transition temperature: 146°C (DSC)

<Example 8>

Nitrogen-containing heterocyclic ring-containing compound (imide compound: mAPDF6-ODPA-PEPA)

A nitrogen-containing heterocyclic ring-containing compound (imide compound) in the form of a yellow powder was obtained in the same manner using mAPDF6 instead of the APDF6 of Example 3.

The physical properties of the obtained nitrogen-containing heterocyclic ring-containing compound (imide compound) are shown below.

FT-IR (KBr, $cm^{-1}$): 2,214 (C≡C), 1,787 (C=O), 1,727 (C=O), 1,373 (C-N), 1,298 (C-F), 745 (imide ring)
Logarithmic viscosity ($\eta_{inh}$): 0.16 dL/g (NMP solution having a 0.5 g/dL concentration, measured at 30°C)
Number average molecular weight (Mn): 4,200 (calculated value 4,000), molecular weight distribution (Mw/Mn): 2.5
5% Weight loss temperature: 528°C (in air), 535°C (in nitrogen) (TGA)
10% Weight loss temperature: 568°C (in air), 568°C (in nitrogen) (TGA)
Carbonization yield: 53% (800°C in nitrogen) (TGA)
Glass transition temperature: 126°C (DSC)

<Example 9>

Nitrogen-containing heterocyclic ring-containing compound (imide compound: mAPDF6-BPDA-PEPA)

A nitrogen-containing heterocyclic ring-containing compound (imide compound) in the form of a yellow powder was obtained in the same manner using mAPDF6 instead of the APDF6 of Example 4.

The physical properties of the obtained nitrogen-containing heterocyclic ring-containing compound (imide compound) are shown below.

FT-IR (KBr, $cm^{-1}$): 2,214 (C≡C), 1,787 (C=O), 1,727 (C=O), 1,373 (C-N), 1,298 (C-F), 745 (imide ring)
Logarithmic viscosity ($\eta_{inh}$): 0.17 dL/g (NMP solution having a 0.5 g/dL concentration, measured at 30°C)
Number average molecular weight (Mn): 5,000 (calculated value 3,900), molecular weight distribution (Mw/Mn) 2.6
5% Weight loss temperature: 526°C (in air), 539°C (in nitrogen) (TGA)
10% Weight loss temperature: 579°C (in air), 575°C (in nitrogen) (TGA)
Carbonization yield: 53% (800°C in nitrogen) (TGA)
Glass transition temperature: 140°C (DSC)

<Example 10>

Crosslinked product from amide compound (6FTA-p-DFBBT-PEPA)

A powder (0.55 g) of an amide compound (6FTA-p-DFBBT-PEPA) having a crosslinkable group was placed in a polyimide film mold cut into a 5 cm × 6 cm mold, and melted by heating at 320°C. Next, a degassing operation was carried out 5 times by applying pressure at 3 MPa. Then, the melted powder was pressed at 320°C for 10 minutes at 5 MPa and at 10 MPa, and further pressed while heating at 370°C for 1 hour at 15 MPa to obtain a crosslinked product film through generation of an imidazole ring by heat dehydration and a crosslinking reaction.
The physical properties of the obtained crosslinked product film are shown below.

FT-IR (KBr, $cm^{-1}$): 1,725 (C=O), 1,495 (C=C), 1,460 (C=N), 1,171 (C-F) Solubility: Insoluble in organic solvents
Glass transition temperature: 297°C (DMA)
5% Weight loss temperature: 493°C (in air), 515°C (in nitrogen) (TGA)
10% Weight loss temperature: 519°C (in air), 544°C (in nitrogen) (TGA)
Carbonization yield: 60% (800°C in nitrogen) (TGA)
Storage elastic modulus (E') : $9\times10^8$ Pa (room temperature to 280°C, glass region), $2\times10^7$ Pa (360 to 400°C, rubber region) (DMA)

<Example 11>

Crosslinked product from nitrogen-containing heterocyclic ring-containing compound (imide compound: APDF6-6FDA-PEPA)

A polyimide film was placed on a SUS plate, a polyimide film from which a 5 cm × 6 cm mold had been cut out was placed thereon, and a powder (0.6 g) of an imide compound (APDF6-6FDA-PEPA) was placed in the mold. A polyimide film and a SUS plate were placed thereon, and the imide compound was melted by heating for 20 minutes at 320°C. Then, degassing was carried out by applying a pressure of 3 MPa for 1 minute, and this operation was repeated 5 times. Pressing was performed at 320°C for 10 minutes at a pressure of 5 MPa, and for further 10 minutes at a pressure of 10 MPa.
Then, the temperature was raised to 370°C, and pressing was performed at 370°C for 1 hour at a pressure of 13 MPa to produce a brown transparent crosslinked product film.
The physical properties of the obtained crosslinked product film are shown below.

FT-IR (film, $cm^{-1}$): 1,785 (C=O), 1,728 (C=O), 1,375 (C-N), 1,258 (C-F), 735 (imide ring)
Solubility: Insoluble in organic solvents
5% Weight loss temperature: 498°C (in air), 514°C (in nitrogen) (TGA)
10% Weight loss temperature: 519°C (in air), 538°C (in nitrogen) (TGA)
Carbonization yield: 52% (800°C in nitrogen) (TGA)
Glass transition temperature: 237°C (DSC), 234°C (DMA), 227°C (TMA)
Storage elastic modulus (E') : $1\times10^9$ Pa (room temperature to 220°C, glass region), $2\times10^7$ Pa (260 to 410°C, rubber region) (DMA)
Thermal expansion coefficient: 73 ppm/°C
Tensile breaking strength: 75 MPa (room temperature) Elongation at break: 6.7% (room temperature)
Tensile elasticity: 2.4 GPa (room temperature) Dielectric constant: 2.61 (20 GHz)
Dielectric tangent: 0.0045 (20 GHz)

<Example 12>

Crosslinked product from nitrogen-containing heterocyclic ring-containing compound (imide compound: APDF6-ODPA-PEPA)

A polyimide film was placed on a SUS plate, a polyimide film from which a 5 cm × 6 cm mold had been cut out was placed thereon, and a powder (0.6 g) of an imide compound (APDF6-ODPA-PEPA) was placed in the mold. A polyimide film and a SUS plate were placed thereon, and the imide compound was melted by heating for 20 minutes at 320°C. Then, degassing was carried out by applying a pressure of 3 MPa for 1 minute, and this operation was repeated 5 times. Pressing was performed at 320°C for 10 minutes at a pressure of 5 MPa, and for further 10 minutes at a pressure of 10 MPa. Then, the temperature was raised to 370°C, and pressing was performed at 370°C for 1 hour at a pressure of 13 MPa to produce a dark-brown opaque crosslinked product film.
The physical properties of the obtained crosslinked product film are shown below.

FT-IR (film, $cm^{-1}$): 1,785 (C=O), 1,727 (C=O), 1,375 (C-N), 1,276 (C-F), 744 (imide ring)
Solubility: Insoluble in organic solvents
5% Weight loss temperature: 494°C (in air), 508°C (in nitrogen) (TGA)
10% Weight loss temperature: 522°C (in air), 537°C (in nitrogen) (TGA)
Carbonization yield: 52% (800°C in nitrogen) (TGA)
Glass transition temperature: 206°C (DSC), 218°C (DMA), 210°C (TMA)
Storage elastic modulus (E') : $1\times10^9$ Pa (room temperature to 200°C, glass region), $2\times10^7$ Pa (260 to 380°C, rubber region) (DMA)
Thermal expansion coefficient: 70 ppm/°C
Dielectric constant: 2.87 (20 GHz)
Dielectric tangent: 0.0037 (20 GHz)

<Example 13>

Crosslinked product from nitrogen-containing heterocyclic ring-containing compound (imide compound: APDF6-BPDA-PEPA)

A brown transparent crosslinked product film was produced in the same manner as in Example 12.
The physical properties of the obtained crosslinked product film are shown below.

FT-IR (film, $cm^{-1}$): 1,785 (C=O), 1,727 (C=O), 1,375 (C-N), 1,276 (C-F), 744 (imide ring)
Solubility: Insoluble in organic solvents
5% Weight loss temperature: 517°C (in air), 533°C (in nitrogen) (TGA)
10% Weight loss temperature: 534°C (in air), 560°C (in nitrogen) (TGA)
Carbonization yield: 56% (800°C in nitrogen) (TGA)
Glass transition temperature: 299°C (DMA), 274°C (TMA)
Thermal expansion coefficient: 66 ppm/°C
Dielectric constant: 2.80 (20 GHz)
Dielectric tangent: 0.0035 (20 GHz)

<Example 14>

Crosslinked product from nitrogen-containing heterocyclic ring-containing compound (imide compound: APHF8-6FDA-PEPA)

A polyimide film was placed on a SUS plate, a polyimide film from which a 5 cm × 6 cm mold had been cut out was placed thereon, and a powder (0.6 g) of an imide compound (APHF8-6FDA-PEPA) was placed in the mold. A polyimide film and a SUS plate were placed thereon, and the imide compound was melted by heating for 20 minutes at 320°C. Then, degassing was carried out by applying a pressure of 3 MPa for 1 minute, and this operation was repeated 5 times. Pressing was performed at 320°C for 10 minutes at a pressure of 5 MPa, and for further 10 minutes at a pressure of 10 MPa. Then, the temperature was raised to 370°C, and pressing was performed at 370°C for 1 hour at a pressure of 10 MPa to produce a brown transparent crosslinked product film.
The physical properties of the obtained crosslinked product film are shown below.

FT-IR (film, $cm^{-1}$): 1,785 (C=O), 1,728 (C=O), 1,375 (C-N), 1,258 (C-F), 735 (imide ring)
Solubility: Insoluble in organic solvents
5% Weight loss temperature: 519°C (in air), 530°C (in nitrogen) (TGA)
10% Weight loss temperature: 546°C (in air), 555°C (in nitrogen) (TGA)
Carbonization yield: 45% (800°C in nitrogen) (TGA)
Glass transition temperature: 193°C (DSC), 193°C (DMA), 205°C (TMA)
Storage elastic modulus (E') : $1\times10^9$ Pa (room temperature to 185°C, glass region), $2\times10^7$ Pa (250 to 410°C, rubber region) (DMA)
Thermal expansion coefficient: 112 ppm/°C
Tensile breaking strength: 46 MPa (room temperature) Elongation at break: 3.3% (room temperature)
Tensile elasticity: 1.5 GPa (room temperature) Dielectric constant: 2.58 (20 GHz)
Dielectric tangent: 0.0022 (20 GHz)

<Example 15>

Crosslinked product from nitrogen-containing heterocyclic ring-containing compound (imide compound: APHF8-ODPA-PEPA)

A polyimide film was placed on a SUS plate, a polyimide film from which a 5 cm × 6 cm mold had been cut out was placed thereon, and a powder (0.6 g) of an imide compound (APHF8-ODPA-PEPA) was placed in the mold. A polyimide film and a SUS plate were placed thereon, and the imide compound was melted by heating for 20 minutes at 320°C. Then, degassing was carried out by applying a pressure of 3 MPa for 1 minute, and this operation was repeated 5 times. Pressing was performed at 320°C for 10 minutes at a pressure of 5 MPa, and for further 10 minutes at a pressure of 10 MPa. Then, the temperature was raised to 370°C, and pressing was performed at 370°C for 1 hour at a pressure of 10 MPa to produce a dark-brown opaque crosslinked product film.
The physical properties of the obtained crosslinked product film are shown below.

FT-IR (film, $cm^{-1}$): 1,785 (C=O), 1,727 (C=O), 1,375 (C-N), 1,276 (C-F), 744 (imide ring)
Solubility: Insoluble in organic solvents
5% Weight loss temperature: 496°C (in air), 515°C (in nitrogen) (TGA)
10% Weight loss temperature: 516°C (in air), 540°C (in nitrogen) (TGA)
Carbonization yield: 51% (800°C in nitrogen) (TGA)
Glass transition temperature: 229°C (DSC), 225°C (DMA), 228°C (TMA)
Storage elastic modulus (E') : $1\times10^9$ Pa (room temperature to 200°C, glass region), $9\times10^7$ Pa (260 to 370°C, rubber region) (DMA)
Thermal expansion coefficient: 82 ppm/°C
Dielectric constant: 2.77 (20 GHz)
Dielectric tangent: 0.0024 (20 GHz)

<Example 16>

Crosslinked product from nitrogen-containing heterocyclic ring-containing compound (imide compound: mAPDF6-6FDA-PEPA)

A brown transparent crosslinked product film was produced in the same manner as in Example 11.
The physical properties of the obtained crosslinked product film are shown below.

FT-IR (film, $cm^{-1}$): 1,785 (C=O), 1,727 (C=O), 1,375 (C-N), 1,276 (C-F), 744 (imide ring)
Solubility: Insoluble in organic solvents
5% Weight loss temperature: 496°C (in air), 493°C (in nitrogen) (TGA)
10% Weight loss temperature: 521°C (in air), 519°C (in nitrogen) (TGA)
Carbonization yield: 49% (800°C in nitrogen) (TGA)
Glass transition temperature: 196°C (DSC), 189°C (DMA), 193°C (TMA)
Storage elastic modulus (E') : $1\times10^9$ Pa (room temperature to 180°C, glass region), $1\times10^7$ Pa (260 to 400°C, rubber region) (DMA)
Thermal expansion coefficient: 95 ppm/°C
Dielectric constant: 2.59 (20 GHz)
Dielectric tangent: 0.0026 (20 GHz)

<Example 17>

Crosslinked product from nitrogen-containing heterocyclic ring-containing compound (imide compound: mAPDF6-ODPA-PEPA)

A brown transparent crosslinked product film was produced in the same manner as in Example 11.
The physical properties of the obtained crosslinked product film are shown below.

FT-IR (film, $cm^{-1}$): 1,785 (C=O), 1,727 (C=O), 1,375 (C-N), 1,276 (C-F), 744 (imide ring)
Solubility: Insoluble in organic solvents
5% Weight loss temperature: 534°C (in air), 535°C (in nitrogen) (TGA)
10% Weight loss temperature: 565°C (in air), 568°C (in nitrogen) (TGA)
Carbonization yield: 53% (800°C in nitrogen) (TGA)
Glass transition temperature: 184°C (DSC), 178°C (DMA), 176°C (TMA)
Storage elastic modulus (E') : $1\times10^9$ Pa (room temperature to 150°C, glass region), $2\times10^7$ Pa (220 to 400°C, rubber region) (DMA)
Thermal expansion coefficient: 93 ppm/°C

<Example 18>

Crosslinked product from nitrogen-containing heterocyclic ring-containing compound (imide compound: mAPDF6-BPDA-PEPA)

A brown transparent crosslinked product film was produced in the same manner as in Example 11.
The physical properties of the obtained crosslinked product film are shown below.

FT-IR (film, $cm^{-1}$): 1,785 (C=O), 1,727 (C=O), 1,375 (C-N), 1,276 (C-F), 744 (imide ring)
Solubility: Insoluble in organic solvents
5% Weight loss temperature: 545°C (in air), 543°C (in nitrogen) (TGA)
10% Weight loss temperature: 576°C (in air), 572°C (in nitrogen) (TGA)
Carbonization yield: 56% (800°C in nitrogen) (TGA)
Glass transition temperature: 207°C (DSC), 203°C (DMA), 199°C (TMA)
Storage elastic modulus (E'): $1\times10^9$ Pa (room temperature to 200°C, glass region), $2\times10^7$ Pa (260 to 400°C, rubber region) (DMA)
Thermal expansion coefficient: 67 ppm/°C
Dielectric constant: 2.69 (20 GHz)
Dielectric tangent: 0.0024 (20 GHz)

<Comparative example 1>

Imide compound: APDF6-6FDA

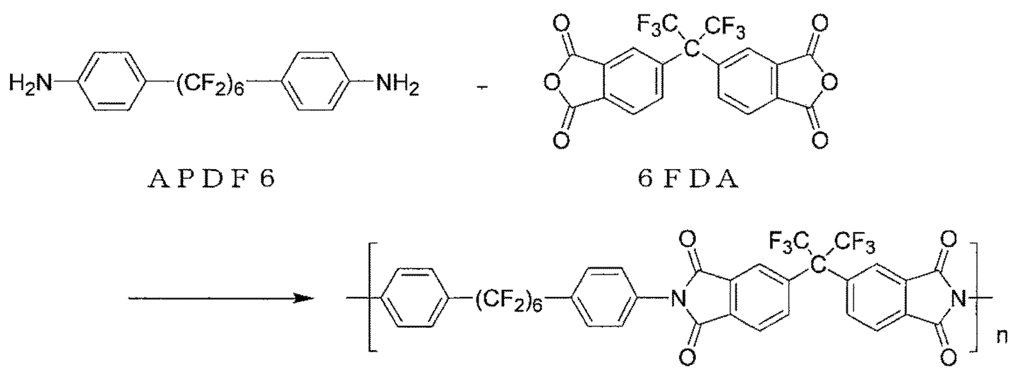
APDF6
6FDA

PI (APDF6−6FDA)

A 100 mL three-necked flask equipped with a stirring rod, a stirring blade, a Dimroth condenser, and a nitrogen introduction tube was heated and dried with a burner, then APDF6 (0.726 g, 1.50 mmol) and distilled NMP (3 mL) were added, and the mixture was stirred to dissolve. Then, 4,4'-(hexafluoroisopylidene)diphthalic acid dianhydride (6FDA) (0.666 g, 1.50 mmol) was added, the temperature was gradually raised, and the mixture was reacted by stirring for 18 hours at 60°C to obtain a solution of the amide compound. The logarithmic viscosity ($\eta_{inh}$) of the amide compound was 0.32 dL/g (NMP solution having a concentration of 0.5 g/dL, measured at 30°C) . The solution was cooled to room temperature, distilled NMP (2 mL) was added, the mixture was stirred to dilute the concentration of the entire solution, and the solution was cast on a glass plate. The cast mixture was dried under reduced pressure for 3 hours at room temperature, then dried under reduced pressure in a program consisting of 6 hours at 60°C, 1 hour at 100°C, 1 hour at 150°C, 1 hour at 200°C, and 1 hour at 250°C to obtain a yellow transparent imide compound film (thickness 22 μm).
The physical properties of the obtained imide compound film are shown below.

FT-IR (KBr, $cm^{-1}$): 1,789 (C=O), 1,725 (C=O), 1,376 (C-N), 1,199 (C-F), 745 (imide ring)
Logarithmic viscosity ($\eta_{inh}$): 0.52 dL/g (NMP solution having a 0.5 g/dL concentration, measured at 30°C)
GPC: Number average molecular weight (Mn) 47,000, molecular weight distribution (Mw/Mn) 2.5
Solubility: Dissolved in NMP, DMAc, DMF, DMI, THF, and chloroform at room temperature
5% Weight loss temperature: 509°C (in air), 524°C (in nitrogen) (TGA)
10% Weight loss temperature: 527°C (in air), 544°C (in nitrogen) (TGA)
Carbonization yield: 52% (800°C in nitrogen) (TGA)
Glass transition temperature: 221°C (DSC), 215°C (DMA), 213°C (TMA)
Storage elastic modulus (E') : $9\times10^8$ Pa (room temperature to 200°C, glass region) (DMA)

## Claims

**1.** An amide compound comprising a repeating unit represented by Formula (1) and a crosslinking site represented by Formula (2) at a molecular end:

Formula (1):

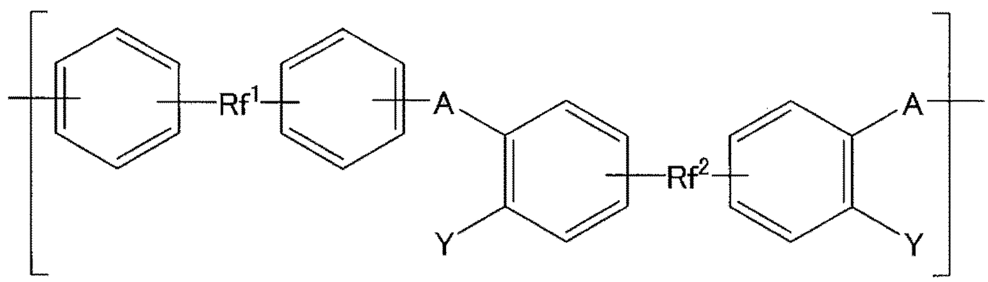

wherein

$Rf^1$ represents a divalent fluorinated organic group,
$Rf^2$ represents a single bond, $-SO_2-$, -O-, -CO-, a divalent non-fluorinated organic group, or a divalent fluorinated organic group,
A represents an amide bond,
each Y independently represents $-COR^1$ or $-NHR^2$,
each $R^1$ independently represents OH, an optionally-substituted linear or branched chain alkoxy group, an optionally-substituted aromatic oxy group, or a halogen atom, and
each $R^2$ independently represents H or a monovalent organic group;

Formula (2): $-C\equiv CX^1$

wherein $X^1$ represents H or a monovalent organic group.

**2.** The amide compound according to claim 1, wherein an average degree of polymerization of the repeating unit represented by Formula (1) is 2 to 10.

**3.** The amide compound according to claim 1 or 2, wherein the repeating unit represented by Formula (1) is a repeating unit represented by Formula (1a):

Formula (1a):

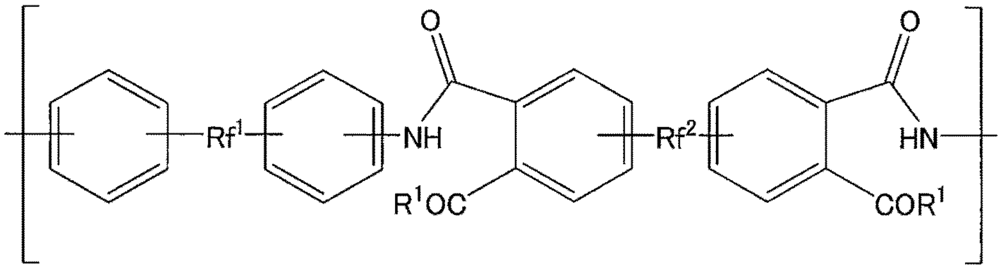

wherein

Rf1 represents a divalent fluorinated organic group,
Rf2 represents a single bond, $-SO_2-$, -O-, -CO-, a divalent non-fluorinated organic group, or a divalent fluorinated organic group, and
each $R^1$ independently represents OH, an optionally-substituted linear or branched chain alkoxy group, an optionally-substituted aromatic oxy group, or a halogen atom.

**4.** The amide compound according to claim 1 or 2, wherein the repeating unit represented by Formula (1) is a repeating unit represented by Formula (1b):

Formula (1b):

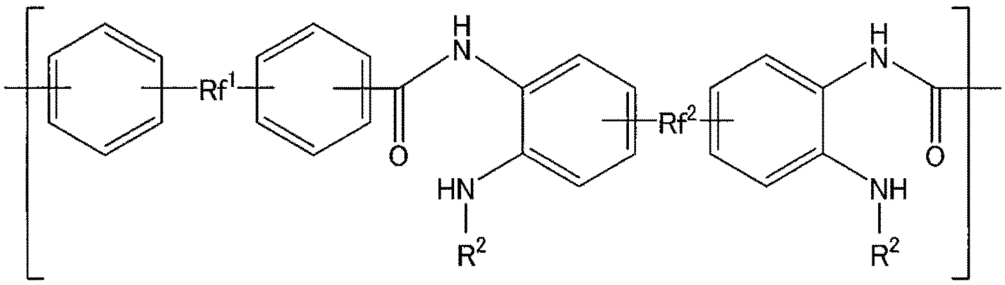

wherein

Rf1 represents a divalent fluorinated organic group,
Rf2 represents a single bond, $-SO_2-$, -O-, -CO-, a divalent non-fluorinated organic group, or a divalent fluorinated organic group, and
each $R^2$ independently represents H or a monovalent organic group.

**5.** The amide compound according to any of claims 1 to 4, wherein Rf1 is a perfluoroalkylene group having 4 or more carbon atoms.

**6.** A nitrogen-containing heterocyclic ring-containing compound comprising a repeating unit represented by Formula (3) and a crosslinking site represented by Formula (2) at a molecular end:

Formula (3):

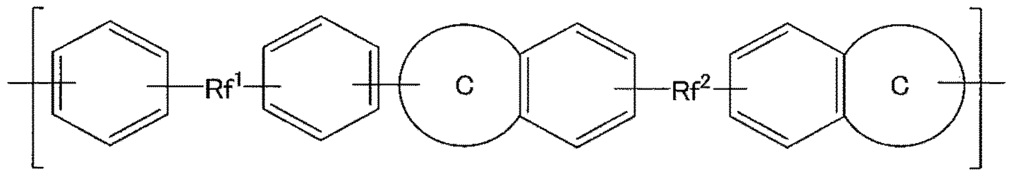

wherein

Rf[1] represents a divalent fluorinated organic group,
Rf[2] represents a single bond, $-SO_2-$, -O-, -CO-, a divalent non-fluorinated organic group, or a divalent fluorinated organic group, and
a ring C represents an optionally-substituted imide ring or benzimidazole ring;

Formula (2): $-C\equiv CX^1$

wherein $X^1$ represents H or a monovalent organic group.

**7.** The nitrogen-containing heterocyclic ring-containing compound according to claim 6, wherein an average degree of polymerization of the repeating unit represented by Formula (3) is 2 to 10.

**8.** The nitrogen-containing heterocyclic ring-containing compound according to claim 6 or 7, wherein the repeating unit represented by Formula (3) is a repeating unit represented by Formula (3a):

Formula (3a):

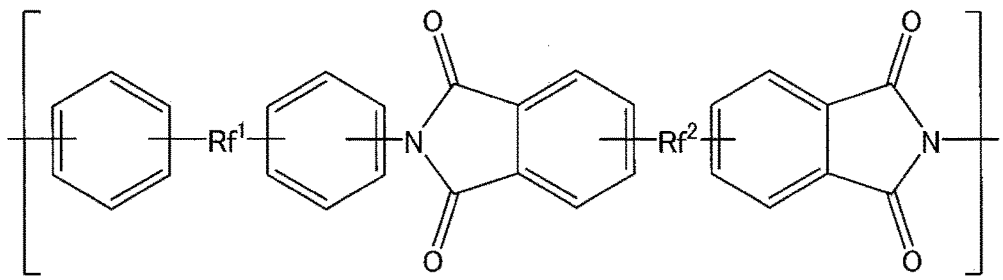

wherein

Rf[1] represents a divalent fluorinated organic group, and
Rf[2] represents a single bond, $-SO_2-$, -O-, -CO-, a divalent non-fluorinated organic group, or a divalent fluorinated organic group.

**9.** The nitrogen-containing heterocyclic ring-containing compound according to claim 6 or 7, wherein the repeating unit represented by Formula (3) is a repeating unit represented by Formula (3b):

Formula (3b):

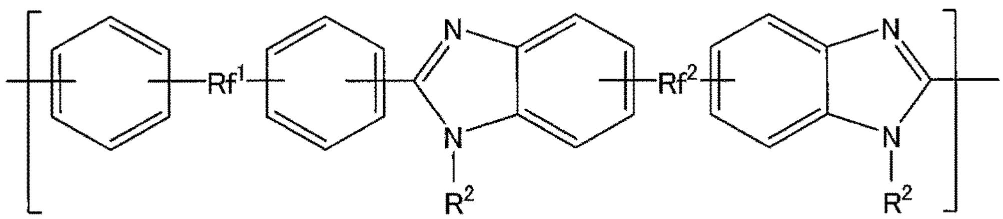

wherein

$Rf^1$ represents a divalent fluorinated organic group,
$Rf^2$ represents a single bond, $-SO_2-$, -O-, -CO-, a divalent non-fluorinated organic group, or a divalent fluorinated organic group, and
each $R^2$ independently represents H or a monovalent organic group.

**10.** The nitrogen-containing heterocyclic ring-containing compound according to any of claims 6 to 9, wherein $Rf^1$ is a perfluoroalkylene group having 4 or more carbon atoms.

**11.** An adhesive comprising the amide compound according to any of claims 1 to 5 or the nitrogen-containing heterocyclic ring-containing compound according to any of claims 6 to 10.

**12.** A coating material comprising the amide compound according to any of claims 1 to 5 or the nitrogen-containing heterocyclic ring-containing compound according to any of claims 6 to 10.

**13.** A crosslinked product obtained by crosslinking the amide compound according to any of claims 1 to 5 or the nitrogen-containing heterocyclic ring-containing compound according to any of claims 6 to 10.

**14.** The crosslinked product according to claim 13, which is a member used in a semiconductor manufacturing apparatus.

## INTERNATIONAL SEARCH REPORT

International application No.
**PCT/JP2021/017951**

### A. CLASSIFICATION OF SUBJECT MATTER

***C08G 73/12***(2006.01)i; ***C08G 73/18***(2006.01)i; ***C09D 179/04***(2006.01)i; ***C09D 179/08***(2006.01)i; ***C09J 179/08***(2006.01)i
FI: C08G73/12; C08G73/18; C09J179/08 A; C09D179/08 A; C09D179/04 A

According to International Patent Classification (IPC) or to both national classification and IPC

### B. FIELDS SEARCHED

Minimum documentation searched (classification system followed by classification symbols)

C08G73/12; C08G73/18; C09D179/04; C09D179/08; C09J179/08

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

CAplus/REGISTRY (STN)

### C. DOCUMENTS CONSIDERED TO BE RELEVANT

| Category* | Citation of document, with indication, where appropriate, of the relevant passages | Relevant to claim No. |
|---|---|---|
| X | JP 06-093118 A (KANEGAFUCHI CHEMICAL INDUSTRY COMPANY LIMITED) 05 April 1994 (1994-04-05) claims, paragraphs [0001], [0002], [0065], examples | 1, 3, 5-6, 8, 10-14 |
| X | WO 89/10946 A1 (HUGHES AIRCRAFT COMPANY) 16 November 1989 (1989-11-16) claims, page 5, line 30 to page 6, line 45, examples | 1-3, 6-8, 11-14 |
| A | US 5344982 A (THE UNITED STATES OF AMERICA AS REPRESENTED BY THE ADMINISTRATOR OF THE NATIONAL AERONAUTICS AND SPACE ADMINISTRATION) 06 September 1994 (1994-09-06) claims, column 1, lines 12-19, column 3, lines 54-58, examples | 1-14 |
| A | PRESTON, P. N. et al. Effect of Monomer Structure on the Cure of PMR Polyimides. HIGH PERFORMANCE POLYMERS., 1990, vol. 2, no. 1, pp. 47-56 entire text | 1-14 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 June 2021** | **06 July 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2021/017951**

| Patent document cited in search report | Publication date (day/month/year) | Patent family member(s) | Publication date (day/month/year) |
|---|---|---|---|
| JP 06-093118 A | 05 April 1994 | (Family: none) | |
| WO 89/10946 A1 | 16 November 1989 | US 4952666 A<br>claims, column 4, line 1 to column 5, line 22, examples<br>EP 419519 A | |
| US 5344982 A | 06 September 1994 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 9208699 A


### Non-patent literature cited in the description


- Newly Revised Latest Polyimides -Basics and Applications. Japan Polyimide / Aromatic Polymer Research Group. NTS Co., Ltd, 25 August 2010, 222-230